# FASCICULE DE BREVET EUROPEEN

(11) **EP 0 907 647 B1**
(45) Date de publication et mention de la délivrance du brevet: **28.11.2001**
(21) Numéro de dépôt: 98913807.8
(22) Date de dépôt: 02.03.1998
(51) Int. Cl.: C07D 323/00, C07D 493/08, G21F 9/00

(54) **CALIX (4)ARENES-COURONNES, LEUR PROCEDE DE PREPARATION ET LEUR UTILISATION POUR L'EXTRACTION SELECTIVE DU CESIUM**
KRONE CALIX-(4)-ARENE, VERFAHREN ZU IHRER HERSTELLUNG UND IHRE VERWENDUNG ZUR SELKTIVEN EXTRAKTION VON CAESIUM
CROWN CALIX (4)ARENES, METHOD OF PREPARATION AND USE FOR SELECTIVE EXTRACTION OF CAESIUM

(30) Priorité: 03.03.1997 FR 9702490
(43) Date de publication de la demande: 14.04.1999
(73) Titulaire: COMMISSARIAT A L'ENERGIE ATOMIQUE, 75015 Paris (FR)
(72) Inventeur: DOZOL, Jean-François, F-04100 Pierrevert (FR); LAMARE, Véronique, F-13100 Aix en Provence (FR); BRESSOT, Christophe, F-75012 Paris (FR); UNGARO, Rocco, I-43040 Vico Fertile (IT); CASNATI, Allessandro, I-43026 San Lazzaro (IT); VICENS, Jacques, F-67000 Strasbourg (FR); ASFARI, Zouhair, F-67000 Strasbourg (FR)
(74) Mandataire: Des Termes, Monique
(86) Numéro de dépôt international: FR9800401
(87) Numéro de publication internationale: WO9839321

(56) Documents cités:
- WO-A-94/12502

## Description

La présente invention a pour objet des calix[4]arènes-couronnes, leur procédé de préparation et leur utilisation pour l'extraction, sélective du césium.

De façon plus précise, elle concerne des calix[4]arènes-couronnes capables d'extraire sélectivement le césium présent à l'état de traces dans des solutions acides ayant ou non des concentrations élevées en cations, telles que les effluents aqueux provenant d'installations de retraitement de combustibles nucléaires usés.

Dans ces effluents, le césium 137 est l'un des produits de fission les plus nocifs du fait de sa longue période (30 ans). Il est donc intéressant de l'éliminer sélectivement des effluents liquides issus des usines de retraitement, en particulier des concentrats d'évaporateurs et des solutions acides possédant ou non une forte salinité due en particulier à la présence de nitrate de sodium.

Etant donné les propriétés chimiques très voisines du sodium et du césium, il est extrêmement difficile d'extraire sélectivement le césium présent dans ces effluents, à une concentration généralement inférieure à 10⁻⁶ mol/l, alors que la concentration en sodium est d'environ 4 mol/l.

Dans les solutions acides de produits de fission, il est également important d'éliminer le ¹³⁵Cs de période 2.10⁶ années, en vue soit de le transmuter soit de l'incorporer dans une matrice spécifique, le césium étant un des éléments les plus mobiles dans un stockage géologique.

### Etat de la technique antérieure

Pour résoudre ce problème, on a envisagé d'extraire le césium au moyen de ligands macrocycliques tels que les para tert-butyl-calixarènes décrits dans US-A-4 477 377. Les para tert-butyl-calixarènes utilisés sont le tétramère, l'hexamère et l'octamère, et les meilleurs résultats sont obtenus avec l'hexamère et l'octamère, le tétramère n'ayant pas une très bonne sélectivité pour séparer le césium du potassium. Cette technique d'extraction du césium est intéressantes mais elle a pour principal inconvénient de ne s'appliquer qu'au traitement de solutions aqueuses basiques, alors que la plupart des effluents provenant du retraitement sont des solutions acides.

Plus récemment, on a envisagé d'utiliser pour cette séparation d'autres calixarènes, en particulier des calixarènes-couronnes, comme il est décrit dans les documents WO-A-94/12502 et WO-A-94/24138.

Selon ces documents, la ou les deux couronnes de calixarènes sont constituées principalement d'enchaînements -C₂H₄-O- qui comportent de 4 à 7 atomes d'oxygène.

Les résultats obtenus avec des couronnes à 6 ou 7 atomes d'oxygène sont satisfaisants pour l'extraction du césium à partir de solutions acides peu chargées en sel. En revanche, lorsqu'on les utilise avec des effluents très chargés un sodium, contenant par exemple 4 mol.l⁻¹ de NaNO₃, leurs performances baissent nettement. Aussi, les recherches ont été poursuivies pour trouver d'autres extractants du même type ayant des performances accrues en milieu acide de haute salinité.

La présente invention a précisément pour objet de nouveaux calixarènes-couronnes qui permettent d'extraire sélectivement le césium à partir de solutions acides et de le séparer du sodium avec une meilleure efficacité.

### Exposé de l'invention

Selon l'invention, les calixarènes sont des calix[4]arènes-couronnes de formule : 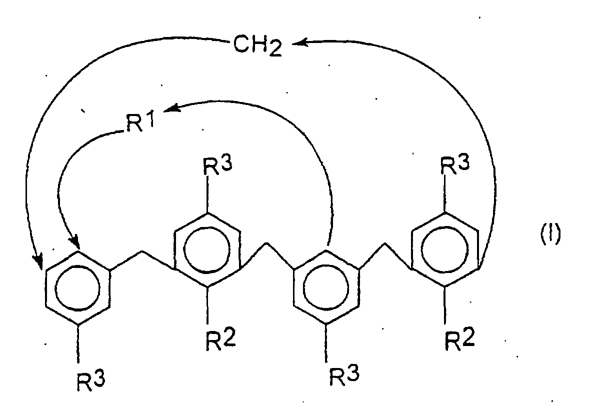 dans laquelle :
- R¹ représente un groupe de formule :

   O-(CH₂CH₂O)ₘ-(XO)ₙ-(CH₂CH₂O)ₚ-(YO)ᵣ-(CH₂CH₂O)ₛ (II),

   où X et Y qui peuvent être identiques ou différents, représentent un groupe arylène ou cycloalkylène, m, n, p, r et s sont des nombres entiers tels que :
   m = s = 1,
   1 ≤ n ≤ 3,
   p = 0 ou 1,
   r = 0 ou 1,
- R² représente le groupe OH ou un groupe alcoxy de 1 à 18 atomes de carbone, les deux R² pouvant être identiques ou différents, ou les deux R² forment ensemble un groupe de formule :

   O-(CH₂CH₂O)ₘ-(XO)ₙ-(CH₂CH₂O)ₚ-(YO)ᵣ-(CH₂CH₂O)ₛ (II),

   dans laquelle X, Y, m, n, p, r et s sont tels que définis ci-dessus ; et
- R³ représente un atome d'hydrogène ou un groupe alkyle de 1 à 3 atomes de carbone,
à condition que, dans le cas où les deux R² forment ensemble un groupe de formule (II), p et r ne soient pas tous deux égaux à 0 lorsque n = 1.

Dans la formule donnée ci-dessus, les groupes alcoxy utilisés pour R² peuvent être linéaires ou ramifiés et ne sont pas nécessairement identique.

Généralement, ils comportent de 1 à 18 atomes de carbone, de préférence de 1 a 12 atomes de carbone.

Dans la formule donnée ci-dessus, on entend par groupe « cycloalkylène », un groupe bivalent dérivé d'un hydrocarbure cyclique par enlèvement d'un atome hydrogène à chacun de deux atomes de carbone du cycle. A titre d'exemple d'un tel groupe, on peut citer le groupe cyclohexylène. Les groupes cycloalkylène utilisés pour X ou Y comportent généralement de 6 à 14 atomes de carbone.

Dans cette formule, on entend par groupe « arylène », un groupe dérivé d'un hydrocarbure aromatique comportant un ou plusieurs noyaux aromatiques ou d'un noyau hétrocyclique comportant un héteroatome tel que 0, S ou N, par enlèvement d'un atome d'hydrogène à chacun de deux atomes de carbone du cycle.

A titre d'exemple de tels groupes, on peut citer les groupes phénylène, naphtylène, benzylène, pyridylène et thiénylène.

Dans les calixarènes de l'invention, la présence d'une ou deux chaînes éthers-couronnes R¹ comportant de 2 à 6 groupes cycliques, par exemple des groupes phénylène, confère à la molécule une plus grande rigidité de la couronne et améliore ses propriétés extractantes et sa sélectivité vis-à-vis cu césium par rapport au sodium, en particulier dans un milieu de forte salinité comme on le verra plus loin.

Selon un premier mode de réalisation de l'invention, le calix[4]arène-couronne de formule (I) comporte une seule chaîne éther-couronne. Dans ce cas, les deux R² de la formule (I) représentent le groupe hydroxyle ou un groupe alcoxy.

Selon un second mode de réalisation de l'invention, le calix[4]arène-couronne de formule (I) comporte deux chaînes éthers-couronnes qui sont de préférence identiques. Dans ce cas, les deux R² forment ensemble un groupe identique à R¹.

Selon l'invention, la ou les chaînes éthers-couronnes représentées par R¹ et répondant aux formules II, IIa ou IIb peuvent comporter de 2 à 6 groupes cycliques saturés ou arylique. On préfère généralement utiliser des groupes aryliques tels que des groupes phénylènes.

De bons résultats pour l'extraction sélective du césium sont obtenus lorsque le calix[4]arène-couronne comprend une ou deux couronnes R¹ de formule : 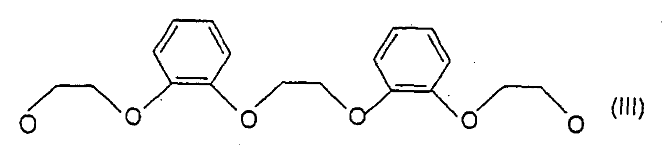 ou 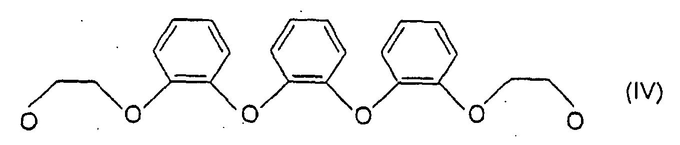 Généralement, dans les calixarènes de l'invention, R³ représente un atome d'hydrogène.

Les calixaranes-couronnes de formule (I) de l'invention peuvent être préparés à partir des calixarènes correspondants par réaction avec un ditosylate comportant le groupe R¹.

Ainsi, les calixarènes-couronnes à une seule couronne, correspondant au premier mode de réalisation de l'invention, peuvent être préparés par réaction d'un calix[4]arène de formule : 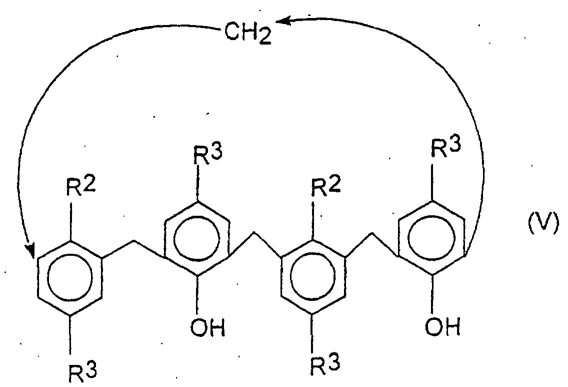 dans laquelle R² et R³ ont les significations données ci-dessus avec un dicosylate de formule (VI) : 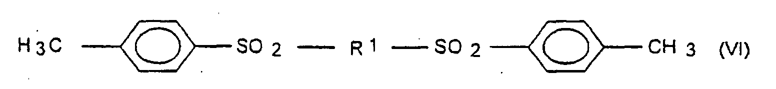 dans laquelle R¹ a la signification donnée ci-dessus.

Les calixarènes-couronnes à deux couronnes correspondant au second mode de réalisation de l'invention de formule (Ia) : 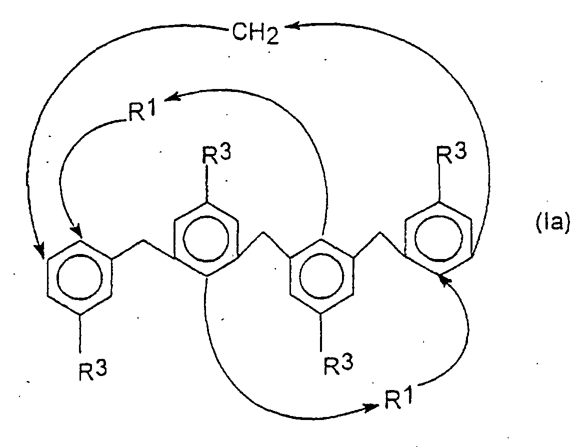 dans laquelle R¹ et R³ sont tels que définis ci-dessus, peuvent être préparés par réaction d'un calix[4]arène de formule : 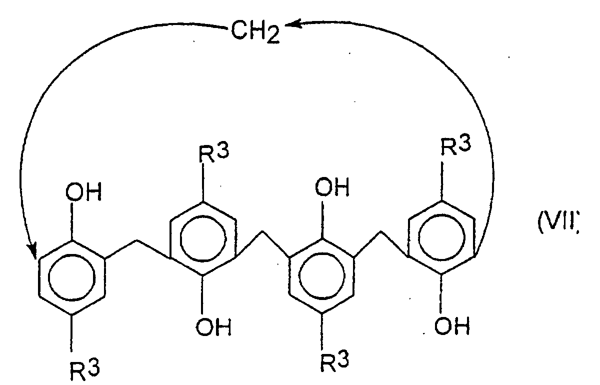 avec un ditosylate de formule : 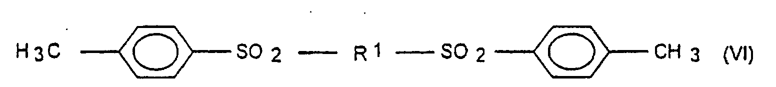 dans laquelle R¹ a la signification donnée ci-dessus.

Pour effectuer cette réaction de formation de la ou des couronnes R¹, on dissout le calixarène de formule (V) ou (VII) dans un solvant approprié, par exemple dans le benzène ou l'acétonitrile, on y ajoute un sel tel que le carbonate de césium et le ditosylate de formule (VI) et on laisse réagir au reflux pendant une durée suffisante pour former la (les) couronnes R¹ reliant deux noyaux benzénique opposés. Après réaction, on évapore le solvant, on reprend le résidu dans de l'acide chlorhydrique et on purifie le produit obtenu par des méthodes classiques.

Le calixarène de formule (V) utilisé comme produit de départ pour la préparation du calix[4]arène-monocouronne peut être préparé à partir du calix[4]arène de formule (VII) par réaction avec un tosylate de formule : 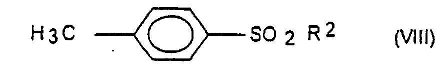 dans laquelle R² a la signification donnée ci-dessus.

Les ditosylates de formule (VI) utilisés comme produits de départ pour la préparation des calixarènes-monocouronnes et bicouronnes peuvent être préparés par des techniques classiques en partant d'un diphénol tel que le catéchol, ou d'un dérivé de diphénol pour établir les liaisons O-CH₂-CH₂-O ou les liaisons : 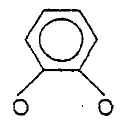 de la chaîne R¹.

Ainsi, dans le cas où R¹ représente le groupe de formule : 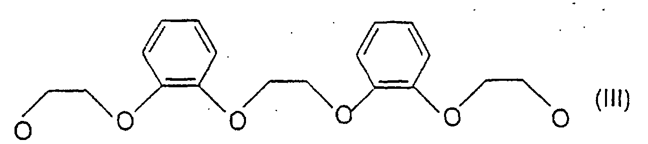 on peut préparer le tosylate en effectuant les étapes suivantes :
- a) synthèse du 2-(2-hydroxyéthoxy)phénol de formule : 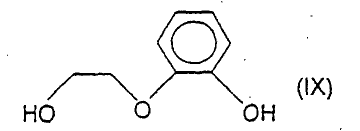 par réaction du catéchol avec du carbonate d'éthylène,
- b) réaction du 2-(2-hydroxyéthoxy) phénol obtenu en a) avec du ditosylate d'éthylène glycol pour obtenir le 1,2-bis[2-(2-hydroxyéthoxy)phénoxy]éthane, et
- c) préparation du tosylate par réaction du 1,2-bis[2-(2-hydroxyéthoxy)phénoxy]éthane avec du chlorure de tosyl de formule :

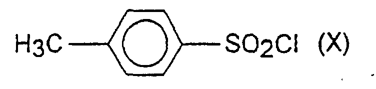

On peut aussi préparer ce tosylate en effectuant les étapes suivantes :
- a) réaction du catéchol avec du 2-chloroéthanol pour obtenir le 2-(2-hydroxyéthoxy)phénol de formule : 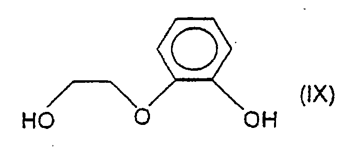
- b) réaction du 2-(2-hydroxyéthoxy)phénol obtenu en a) avec du 1,2dibromoéthane pour obtenir le 1,2-bis[2-(2-hydroxyéthoxy)phenoxyléthane, et
- c) préparation du tosylate par réaction du 1,2-bis[2-(2-hydroxyéthoxy)phénoxy]éthane avec du chlorure de tosyle de formule (X) :

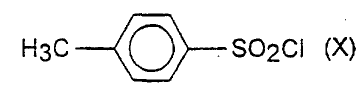

Dans le cas où R¹ représente le groupe de formule (IV), on peut préparer le tosylate en effectuant les étapes suivantes :
- a) préparation du diphénol de formule : 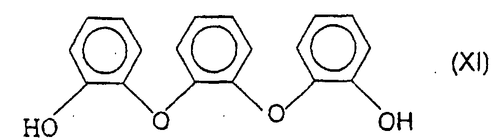
- b) réaction du diphénol avec du ditosylate d'éthylène glycol.

On peut aussi préparer ce tosylate en effectuant les étapes suivantes :
- a) préparation du diphénol de formule : 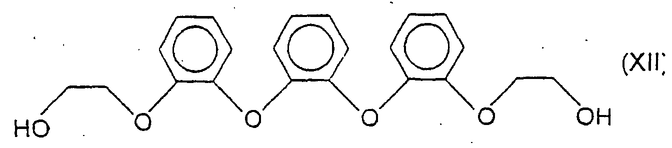
- b) réaction du diphénol avec du chlorure de tosyle :

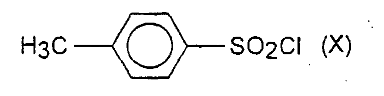

Les calix[4]arènes-couronnes de formule (I) décrits précédemment peuvent être utilisés en particulier pour l'extraction sélective du césium présent dans des solutions aqueuses, notamment des solutions acides chargées ou non en sodium telles que les solutions de dissolution et les effluents aqueux provenant d'installation de retraitement de combustibles nucléaires usés.

Pour l'extraction du césium, les solutions aqueuses de départ peuvent être des solutions acides, par exemple des solutions nitriques contenant de 10⁻³ à 7 mol/l d'acide nitrique.

Pour réaliser cette extraction, on met en contact la solution aqueuse de départ, dite première solution aqueuse, contenant le césium avec une phase liquide immiscible comprenant le calix[4]arène-couronne, puis on sépare de la première solution aqueuse la phase liquide immiscible.

On peut ensuite récupérer le césium dans une seconde solution aqueuse par mise en contact de la phase liquide immiscible ayant extrait le césium avec cette seconde solution aqueuse de réextraction. Cette dernière peut être constituée par de l'eau distillée et désionisée.

Pour mettre en oeuvre ce procédé, on peut effectuer la mise en contact de la première solution et de la deuxième solution aqueuse avec la phase liquide immiscible dans des installations classiques d'extraction liquide-liquide telles que des mélangeurs-décanteurs, des colonnes pulsées, etc.

On peut aussi effectuer cette mise en contact en disposant la phase liquide immiscible comprenant le calix[4]arène-couronne sous la forme d'une membrane liquide comportant deux surfaces opposées, la première solution aqueuse de départ contenant le césium étant au contact d'une des surfaces de la membrane, et en recueillant le césium extrait par la membrane liquide dans la seconde solution aqueuse de réextraction au contact de la surface opposée de la membrane.

Pour former la phase liquide immiscible, on dissout le calix[4]arène-couronne dans un solvant approprié.

A titre d'exemple de solvants utilisables, on peut citer les alkylbenzènes et les nitrophénylalkyl éthers.

De préférence, on utilise comme solvant un éther tel que l'ortho-nitrophénylhexyléther et l'ortho-nitrophényloctyléther.

La concentration en calix[4]arène-couronne de la phase liquide immiscible, dépend en particulier du solvant utilisé. On peut utiliser des concentrations allant de 10⁻⁴ à 5.10⁻¹ mol/l, par exemple une concentration de 10⁻² mol/l.

Selon l'invention, la phase immiscible peut être aussi une phase solide. Dans le cas d'une phase solide, celle-ci peut être imprégnée d'un ou plusieurs calixarènes de l'invention, à l'état pur ou en solution dans un solvant appropriée. On peut aussi utiliser une phase solide sur laquelle est greffé le(s) calixarène(s) de l'invention.

Dans le cas où l'on utilise une phase immiscible solide, celle-ci peut être constituée par un support solide sur lequel est immobilisé le calixarène, qui peut former une membrane liquide supportée, constituée par une membrane microporeuse jouant le rôle de support, dont les pores sont remplis de calixarène(s) en solution dans un solvant organique approprié.

Cette membrane microporeuse peut être réalisée en polypropylène, en fluorure de polyvinylidène ou en polytétrafluoréthylène, et elle peut servir de séparation entre un premier compartiment dans lequel se trouve la première solution aqueuse à traiter et un deuxième compartiment dans lequel se trouve la seconde solution aqueuse de réextraction.

Pour obtenir une bonne extraction avec les membranes liquides supportées, il est avantageux d'utiliser des membranes ayant une faible épaisseur, une grande porosité et un petit diamètre de pores. Ces membranes peuvent être utilisées sous la forme de modules tels que les modules d'ultra ou de microfiltration à membranes planes ou à fibres creuses, qui permettent de traiter des débits importants de fluide.

D'autres caractéristiques et avantages de l'invention apparaîtront mieux à la lecture des exemples suivants donnés bien entendu à titre illustratif et non limitatif en référence aux dessins annexés.

### Brève description des dessins

La figure 1 (1A et 1B) illustre deux schémas de synthèse de calixarènes conformes à l'invention. Sur cette figure, la voie A correspond à un calixarène-monocouronne et la voie B correspond à un calixarène bicouronne.

La figure 2 (2A et 2B) illustre le schéma de synthèse d'une autre calixarène-monocouronne conforme à l'invention.

La figure 3 (3A et 3B) illustre le schéma de synthèse d'un autre calixarène-biscouronne conforme à l'invention.

Les figures 4 et 5 sont des graphiques illustrant l'évolution de la concentration en césium de la solution de départ en fonction du temps (en heures), lors de l'extraction du césium par un calixarène-bis couronne (figure 4) et par un calixarène-monocouronne (figure 5).

### Exposé détaillé des modes de réalisation

Les exemples qui suivent illustrent la préparation de calix[4]arènes conformes à l'invention et leur utilisation pour l'extraction du césium à partir de solutions aqueuses acides chargées ou non en sel.

Les exemples 1 et 2 illustrent la préparation de calixarènes monoccuronnes, et les exemples 3 et 4 illustrent la préparation de calixarènes bis-couronnes.

### Exemple 1 : Préparation du 25, 27-dioctyloxy-calix[4]arène-dibenzo-couronne-6 (composé 7).

Ce calixarène répond à la formule (I) de l'invention avec R¹ représentant le groupe de formule (III), les deux R² représentant le groupe octoxy, et les R³ étant un atome d'hydrogène.

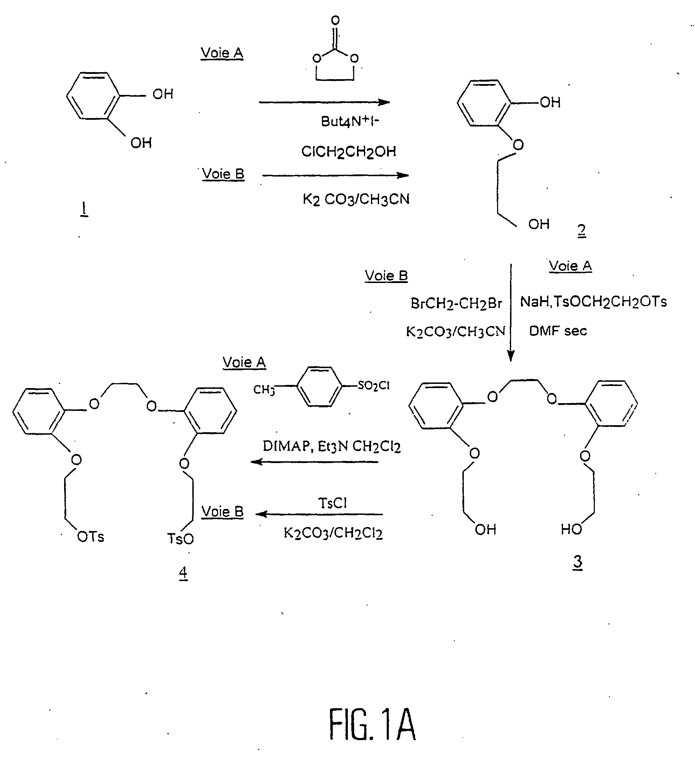

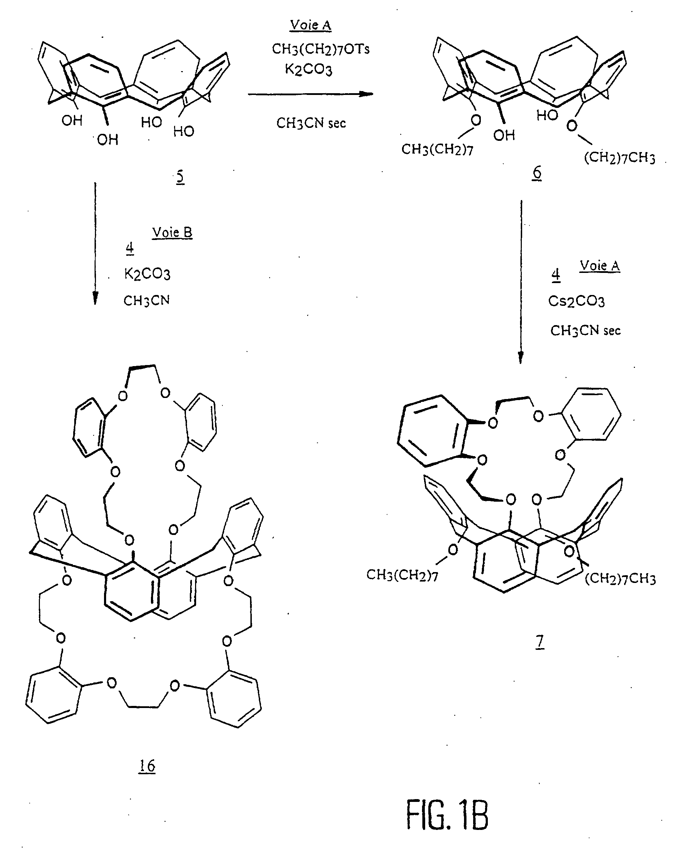

Cette synthèse est effectuée selon le schéma réactionnel illustré sur la figure 1 (voie A).

Les étapes a) et b) qui suivent utilisent une méthode de synthèse adaptée à partir de Yamaguchi, K., Negi, S., Kozakai, S., Nagano, R., Kuboniwa, H., Hirao, A., Nakahama, S., Yamazaki, N., *Bull*. *Chem. Soc.* Jpn. ; 61, 2047-2054, 1988.

### a) Synthèse de 2-(2-hydroxyéthoxy)phénol (composé 2).

On chauffe à 150°C, un mélange de catéchol 1 (22 g, 0,2 mol), de carbonate d'éthylène (17,6 g, 0,2 mol) et d'iodure de tétrabutyl ammonium (2,4 g, 6,5 mol) pendant environ 1 heure jusqu'à ce que le dégagement de CO₂ s'arrête.

On purifie le mélange par chromatographie sur colonne de silice en utilisant comme éluant un mélange hexane-acétate d'éthyle (1 : 1) et l'on obtient un solide blanc. Par recristallisation dans l'eau, on obtient 4,1 g de composé 2 pur, ce qui correspond à un rendement de 46 %.

Les caractéristiques du composé **2** sont les suivantes :
Point de fusion F : 100-101°C.
¹H RMN (CDCl₃) δ (ppm) : 6,95-6,80 (m, 5H, ArOH et ArH), 4,13-4,10 (m, 2H, ArOCH₂),4,0-3,97(m, 3H, OCH₂CH₂OH).
Spectrométrie de masse (CI) : m/e = 155 (M⁺).

### b) Synthèse du 1,2-bis[2-(2-hydroxyéthoxy)-phénoxy]éthane (composé 3).

On agite à la température ambiante, pendant 30 minutes un mélange du composé **2** (2 g, 13 mmol) et de NaH (0,32 g, 13 mmol) dans 8 ml de diméthylformamide (DMF) sec. A cette solution, on ajoute goutte à goutte le ditosylate d'éthyléneglycol (2,4 g, 6,4 mmol) dissous dans 25 ml de DMF sec et on agite le mélange à température ambiante pendant 36 heures. Après élimination du DMF sous vide, on dissout le résidu dans du dichlorométhane et on le lave avec de l'eau. La couche organique, séchée sur sulfate de sodium, est évaporée. Par recristallisation à partir d'un mélange éthanol-eau (1 : 1), on obtient 1,71 g du composé 3 pur, ce qui correspond à un rendement de 81 %.

Les caractéristiques de ce composé sont les suivantes:
F = 75-77°C
¹H RMN(CDCl₃)δ (ppm) : 6,97-6,89 (m, 8H, ArH), 4,39 (s, 4H, ArOCH₂CH₂OAr), 4,34 (s, 2H, OH), 4,1(t, J = 7 Hz, 4H, ArOCH₂CH₂OH), 3,87 (t, J = 7 Hz, 4H, ArOCH₂CH₂OH).
Spectrométrie de masse : m/e 335 (M⁺).

### c) Synthèse du 1,2-bis[2-(2-hydroxyéthoxy)-phénoxy]éthane, bis(p-toluènesulfonate) (composé 4).

Pour cette synthèse, on utilise une méthode adaptée à partir de Weber, E., J. Org. Chem. ; 47, 1982, pages 3478-3486.

On refroidit à 0°C, un mélange du composé **3** (2 g, 5,9 mmol), et de triéthylamine(1,7 ml, 12mmol) dans 32 ml de dichlorométhane. A cette solution, on ajoute du chlorure de tosyle (2,3 g, 12 mmol) et de la 4-diméthylaminopyridine (7,3 mg, 0,06 mmol).

Après 3 heures, on extrait le mélange avec 50 ml de HCl à 10 %. On sèche la couche organique sur du sulfate de sodium et on évapore sous vide. Par recristallisation à partir de méthanol, on obtient 2,3 g de composé **4** pur, ce qui correspond à un rendement de 60 %. Les caractéristiques de ce composé sont les suivantes :
F : 91 - 93°C.
¹H RMN(CDCl₃)δ (ppm) 7,75 (d, J = 8 Hz, 2H, ArOCH₃), 7,27 (d, J = 8, 2H, ArCH₃), 6,86-6,81 (m, 8H, ArH), 4,34 (s, 4H, ArOCH₂CH₂OAr), 4,19-4,16 (m, 8H, TSOCH₂CH₂O), 2,39 (s, 6H, CH₃Ar).
Spectrométrie de masse (CI) : m/e 642 (M⁺).

### d) Synthèse du composé 7.

On porte au reflux pendant 30 minutes sous atmosphère d'azote, une solution du calix[4]arène **5** (1,0 g, 2,36 mmol) et de carbonate de potassium (0,48 g, 3,53 mmol) dans 15 ml d'acétonitrile sec.

Après addition de tosylate d'octyle (2,01 g, 7,07 mmol), on porte le mélange au reflux pendant 48 heures. On élimine alors l'acétonitrile sous pression réduite et on traite le résidu avec 50 ml de HCl à 10 % et 50 ml de diclorométhane. On sépare la couche organique, on la lave deux fois avec de l'eau distillée, on la sèche sur sulfate de magnésium, puis on élimine le dichlorométhane par distillation. On ajoute alors de l'éthanol et on sépare le précipité par filtration. On obtient ainsi 1,3 g d'un solide blanc (rendement de 85 %) correspondant au 25,27-dioctoxycalix[4]arène **6**.

On le sèche sous vide poussé pendant quelques heures, on le dissout dans 310 ml d'acétonitrile et on ajoute un excès de carbonate de césium (2,64 g, 8,1 mmol) et le composé **4** (1,3 g, 2,02 mmol) sous atmosphère d'azote. On porte le mélange réactionnel au reflux pendant 16 heures, puis on le traite par HCl à 10 % et on effectue des lavages et une séparation comme précédemment dans le cas du composé **6**.

On obtient ainsi 1,07 g de composé **7**, le 25,27-dioctoxycalix[4]arène-dibenzo-couronne-6 (rendement de 56 %) à partir du résidu huileux par chromatographie sur colonne de silice en utilisant comme éluant un mélange de tétrahydrorurane THF et d'hexane (1:9) et cristallisation à partir de méthanol.

Les caractéristiques du composé **7** sont les suivantes :
F : 130°C
¹H RMN(CDCl₃)δ(ppm) : 7,10-6,99(m, 12H, ArH), 6,80 (t, J = 6,5 Hz, 2H, ArH para), 6,78 (d, J = 6,5 Hz, 4H, ArH meta), 6,58 (t, J = 6,5 Hz, 2H, ArH para), 4,37 (s, 4H, ArOCH₂CH₂OAr), 3,77(s, 8H, ArCH₂Ar), 3,63 (t, J = 6 Hz, 4H, ArOCH₂CH₂OArO), 3,47 (t, J = 6 Hz, 4H, ArOCH₂CH₂OArO), 3,36 (t, J = 6 Hz, 4H, ArOCH₂R), 1,36-1,1(m, 24H, OCH₂(CH₂)₆CH₃), 0,92 (t, J.= 6,5 Hz, 6H, -CH₃).
Spectométrie de masse (CI) : m/e = 948 (M⁺)

### Exemple 2 : Synthèse du 1,3-dioctoxy calix[4]arène-tribenzo-couronne-6 (composé 15).

Ce composé répond à la formule (I) de l'invention avec R¹ représentant le groupe de formule (IV), les deux R² représentant le groupe octoxy et R³ représentant un atome d'hydrogène.

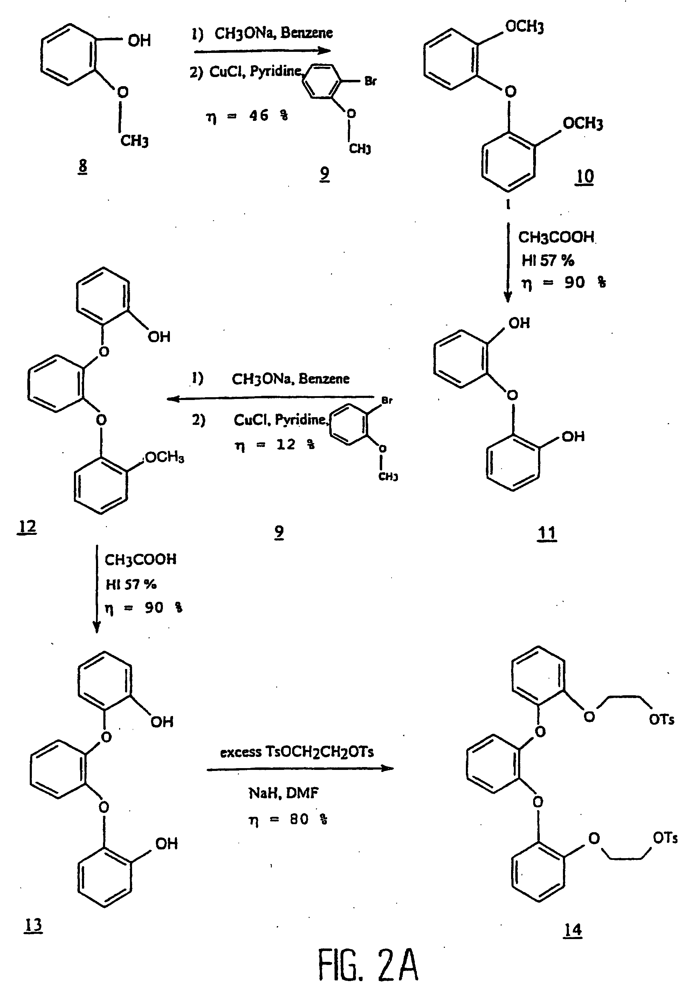

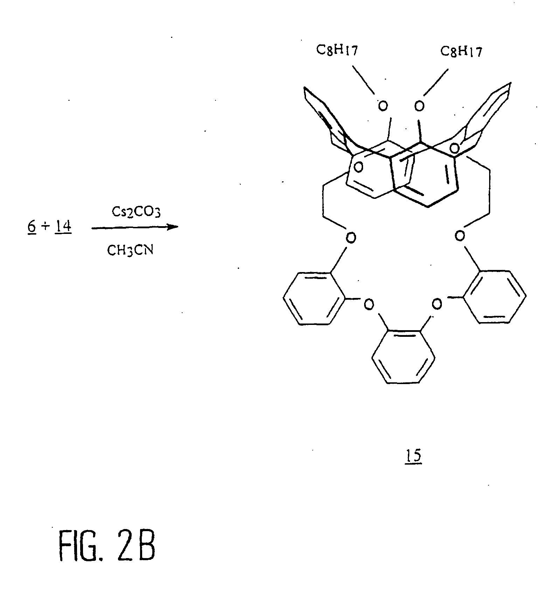

Cette synthèse correspond au schéma illustré sur la figure 2.

Pour cette synthèse, on suit le même mode opératoire que dans l'exemple 1, en faisant réagir comme dans l'étape d) de l'exemple 1, le ditosylate **14** correspondant au groupe R¹ de formule (IV) avec le composé 6, soit le 25, 27-dioctoxy calix[4]arène.

Le ditosylate est obtenu conformément au schéma de synthèse illustré sur la figure 2. Cette synthèse est réalisée en suivant la méthode décrite par D. E. Kime, J. K. Norymbersky, J. Chem. Soc. Perkin Trans 1, 1977, page 1048, pour préparer le composé **13.**

Le composé **13** est traité ensuite avec un excès de di-p-toluène sulfonate d'éthylène-glycol dans DMF sec avec de l'hydrure de sodium pendant 8 heures. On élimine le DMF sous pression réduite, et on purifie le composé **14** par chromatographie sur colonne.

On fait ensuite réagir ce composé **14** avec le calix[4]arène **6** de l'exemple 1 en suivant le même mode opératoire que dans l'exemple 1.

Les caractéristiques du composé **15** ainsi obtenu sont les suivantes :
¹H RMN (CDCl₃) δ (ppm) : 7,22-6,75 (m, 24H, ArH), 3,78 (s, 8H, ArCH₂Ar), 3,65 (t, J = 6 Hz, ArOCH₂CH₂OAr), 3,43 (t, J = 6 Hz, ArOCH₂CH₂OAr), 3,36 (t, J = 6 Hz, 4H, ArOCH₂(CH₂)₆CH₃), 1,3-1,1 (m, 24H, OCH₂(CH₂)₆CH₃), 0,90 (t, J = 6,5 Hz, 6H, OCH₂(CH₂)₆CH₃).
Spectrométrie de masse : (CI) : m/e 994,5 (M⁺).

### Exemple 3 : Synthèse du calix[4]arène-bis-dibenzo-couronne-6, composé 16.

Ce composé répond à la formule (I) de l'invention avec R¹ représentant le groupe de formule (III), les deux R² formant ensemble le groupe de formule (III) et R¹ représentant un atome d'hydrogène.

Pour cette synthèse, on suit le schéma réactionnel représenté sur la figure 1, voie B. Ainsi, on part du catéchol composé 1 utilisé comme produit de départ dans l'exemple 1, et on le transforme en composé **2**, puis en composé **3** et en composé **4** en opérant d'une façon légèrement différente. Ensuite, on prépare calix[4]-bis-couronne par réaction du composé **4** avec le composé **5**.

### a) Préparation du composé 2

Dans un ballon de 1 l, on introduit 33,03 g (300 mmol) de catéchol **1**, 24,15 g (300 mmol) de 2-chloroéthanol, 20,75 g (150 mmol) de K₂CO₃ et 750 ml d'acétonitrile. Le mélange réactionnel est mis sous reflux pendant 48 heures. On le laisse ensuite revenir à la température ambiante, puis on filtre la solution et on la concentre. Après purification par chromatographie sur colonne (CHCl₃/acétone : 80/20), le composé **2** est récupéré sous la forme de solide blanc (18,5 g) ; rendement de 40 %.
Spectre ¹H RMN dans CDCl₃ à 200 MHz, δ en ppm
δ = 6,99 à 6,78 (m, 4H, Ar)
δ = 4,16 à 4,11 (m, 2H, OCH₂CH₂)
δ = 4,03 à 3,98 (m, 2H, OCH₂CH₂OH)
δ = 2,79 (s large, 2H, OH).

### b) Préparation du composé 3

Dans un ballon de 1000 ml, on introduit 7,71 g (50 mmol) de composé **2**, 69,1 g (500 mmol) de K₂CO₃, 5,64 g (30 mmol) de 1,2 dibromoéthane et 750 ml de CH₃CN. Le mélange réactionnel est mis sous reflux pendant 4 jours. Après l'avoir laissé à la température ambiante, le solvant est évaporé à sec. Le résidu alors obtenu est solubilisé dans du CH₂Cl₂ et du K₂CO₃ neutralisé avec HCl 1N. La phase organique est séchée sur sulfate de sodium, puis concentrée. Après purification par chromatograpie sur colonne (CH₂Cl₂/Acétone : 85/15) ; le composé **3** est récupéré sous forme d'un solide blanc (4,2 g)
Rendement : 25 %
Spectre ¹H RMN dans CDCl₃ à 200 MHz, δ en ppm
δ = 6,97 (s, 8H, Ar)
δ = 4,40 à 4,34 (m, 6H, OCH₂CH₂O et OH)
δ = 4,14 à 4,10 (m, 4H, OCH₂CH₂)
δ = 3,89 à 3,86 (m, 4H, OCH₂CH₂)

### c) Préparation du composé 4

Dans un bicol de 100 ml, on introduit 2,0 g (6 mmol) de composé **3**, 2,3 g (12 mmol) de chlorure de tosyle et 70 ml de dichloroéthane. Le mélange réactionnel est placé dans un bain de glace à 0°C, puis 2,3 g (22 mmol) de triéthylamine sont ajoutés goutte à goutte. Après 48 heures de réaction à température ambiante, la triéthylamine est neutralisée avec une solution d'acide HCl 1N. La phase organique est séchée sur sulfate de sodium, puis concentrée pour donner un solide beige. Après purification par chromatographie sur colonne (CH₂Cl₂/acétone : 95/5), le produit ditosylé est récupéré sous forme d'un solide blanc (2,4 g).
Rendement : 62 %
Spectre ¹H RMN dans CDCl₃ à 200 MHz, δ en ppm
δ = 7,77 (d, 4H, J = 8,5 Hz, système AB OTS)
δ = 7,28 (d, 4H, J = 8,5 Hz, système AB OTS)
δ = 6,97 à 6,81 (m, 8H, Ar)
δ = 4,31 à 4,27 (m, 8H, OCH₂CH₂O)
δ = 4,20 à 4,16 (m, 4H, OCH₂CH₂O)
δ = 2,40 (s, 6H, ArCH₃)

### d) Préparation du composé 16

Dans un ballon de 250 ml, on introduit 0,425 g (1 mmol) de calix[4]arène (composé **5**), 1,380 g (10 mmol) de K₂CO₃ dans 100 ml de CH₃CN. On agite à température ambiante pendant 4 heures, puis on rajoute 0,643 g (1 mmol) de ditosylate **4** et on chauffe sous reflux pendant 7 jours. La même quantité de K₂CO₃ et de ditosylate est encore une fois ajoutée à la solution et le mélange réactionnel est porté au reflux pendant 7 jours supplémentaires. Après 14 jours de reflux, le solvant est évaporé à sec. Le résidu obtenu est solubilisé dans le dichlorométhane et K₂CO₃ neutralisé avec du HCl 1N. La phase organique est séchée sur sulfate de sodium, puis concentrée. Après purification par chromatographie sur colonne (CH₂Cl₂/acetone 90/10), le composé **16** dibenzo-bis- couronne-6 est récupéré sous forme d'un solide blanc (850 mg).
Rendement : 83 %
Spectre ¹H RMN dans CDCl₃ à 200 MHz, δ en ppm
δ = 7,14 à 7,08 (m, 8H, Ar)
δ = 6,96 à 6,89 (m, 8H, Ar)
δ = 6,72 (d, 8H, J = 7 Hz, H meta du Ar)
δ = 6,58 (t, 4H, J = 7 Hz, H para du Ar)
δ = 4,39 (s, 8H, OCH₂CH₂O)
δ = 3,74 (s, 8H, ArCH₂-Ar)
δ = 3,49 (m, 16H, OCH₂CH₂O)
Spectre de masse FAB positif m/z = 1021,5 (100 %)

### Exemple 4 : Synthèse du calix[4]arène-bis-couronne-6 (composé 17).

Ce calixarène répond à la formule (I) de l'invention avec R¹ représentant le groupe de formule (IV), les deux R² formant ensemble le même groupe de formule (IV), et R³ représentant un atome d'hydrogène.

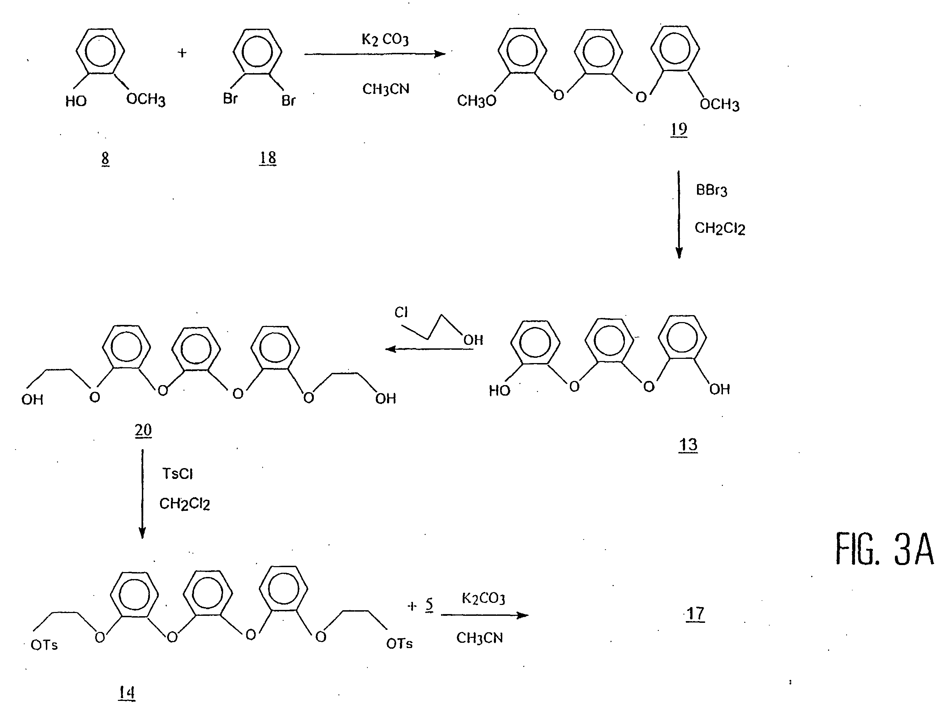

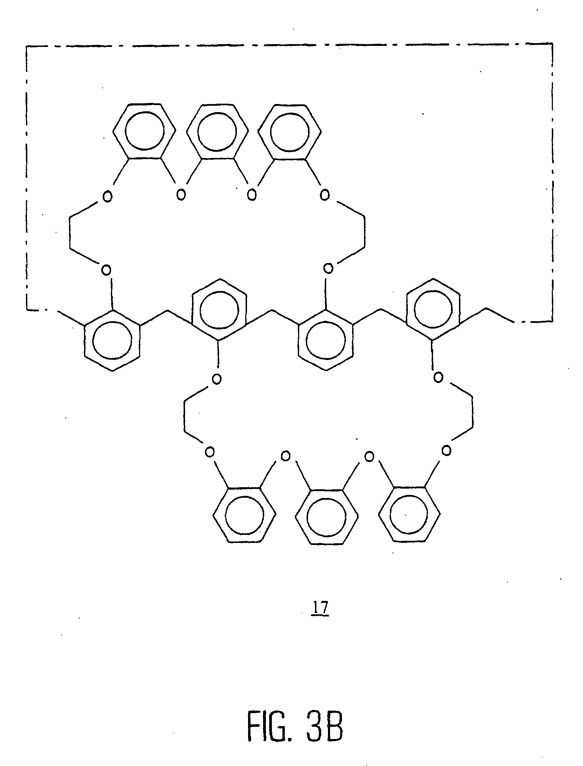

Pour cette synthèse, on suit le schéma de synthèse donné sur la figure 3. On prépare tout d'abord le ditosylate à partir du composé **8** utilisé dans l'exemple 2, mais en effectuant des étapes un peu différentes.

### a) Préparation du composé 19

Dans un ballon de 500 ml, on introduit 4,47 g (36 mmol) de Guaiacol **8** ou 2 méthoxyphénol, 24,88 g (180 mmol) de K₂CO₃ et 350 ml d'acétonitrile. Après avoir agité la solution pendant 2 heures à la température ambiante, on ajoute goutte à goutte 4,25 g (18 mmol) de 1,2-dibromobenzène **18** et on chauffe le mélange réactionnel sous reflux pendant 4 jours. Après avoir laissé le mélange réactionnel revenir à la température ambiante, le solvant est évaporé à sec. Le résidu alors obtenu est solubilisé dans le dichlorométhane et K₂CO₃ neutralisé avec de l'acide chlorhydrique HCl 1N. La phase organique est séchée sur sulfate de sodium, puis concentrée. Après purification ,par chromatographie sur colonne (CH₂Cl₂), le produit est récupéré sous forme d'un solide jaune (3,45 g).
Rendement : 30 %
Spectre ¹H RMN dans CDCl₃ à 200 MHz, δ en ppm
δ = 6,95 à 6,85 (m, 12H, Ar)
δ = 3,89 (s, 6H, CH₃).

### b) Préparation du composé 13

Dans un bicol de 500 ml, on introduit 10 g (40 mmol) de tribromure de bore BBr₃ et 300 ml de dichlorométhane. Après avoir agité la solution pendant 30 minutes à -60°C, on ajoute goutte à goutte 3,22 g (10 mmol) de 2,2-[1,2 phénylènedi(oxy) diphényl diméthyléther] **19** dans 100 ml de CH₂Cl₂ en maintenant la température à -60°C. Après 10 heures de réaction, l'excès de BBr₃ est détruit en ajoutant du méthanol. Le résidu obtenu est évaporé à sec, puis solubilisé dans du dichlorométhane. La phase organique est lavée avec une solution de MaHCO₃ à 5 %, puis séchée sur Na₂SO₄ et concentrée. Un solide blanc est récupéré par recristallisation dans du n-heptane (2,6 g)
Rendement : 88 %
Spectre ¹H RMN dans CDCl₃ à 200 'MHz, δ en ppm
δ = 6,97 à 6,88 (m, 12H, Ar)
δ = 6,58 (s, 2H, CH₃).

### c) Préparation du composé 20

Dans un ballon de 500 ml, on introduit 2,35 g (8 mmol) de 2,2'-[1,2-phénylènedi(oxy)diphénol]**13**, 2,58 g (32 mmol) de 2-chloroéthanol, 11 g (80 mmol) de K₂CO₃ et 200 ml d'acétonitrile. Le mélange réactionnel est mis sous reflux pendant 3 jours. Après l'avoir laissé revenir à température ambiante, il est évaporé à sec. Le résidu obtenu est solubilisé dans le dichlorométhane et K₂CO₃. neutralisé avec de l'acide chlorhydrique HCl 1N. La phase organique est séchée sur sulfate de sodium, puis concentrée. Après purification par chromatographie sur colonne (CH₂Cl₂ : acétone - 90 : 10), le produit **20** est récupéré sous forme de solide blanc (1,9 g)
Rendement : 62 %.
Spectre ¹H RMN dans CDCl₃ à 200 MHz, δ en ppm
δ = 6,87 à 6,74 (m, 12H, Ar)
δ = 4,32 (s large, 2H, OH)
δ = 4,07 à 4,02 (m, 4H, OCH₂CH₂)
δ = 3,74 à 3,69 (m, 4H, OCH₂CH₂)

### d) Préparation du ditosylate 14

Dans un bicol de 100 ml, on introduit 1,8 g (4,7 mmol) de diéthylène glycol triphénol **20**, 1,8 g (9,4 mmol) de chlorure de tosyle et 60 ml de dichloroéthane.

Le mélange réactionnel est placé dans un bain de glace à 0°C, puis 2,3 g (22 mmol) de triéthylamine sont ajoutés goutte à goutte. Après 48 heures de réaction à température ambiante, la triéthylamine est neutralisée avec une solution d'acide HCl 1N. La phase organique est séchée sur sulfate de sodium, puis concentrée pour donner un solide. Après purification par chromatographie sur colonne (CH₂Cl₂ : acétone - 98 : 2), le produit ditosylé **14** est récupéré sous forme d'un solide blanc (1,95 g).
Rendement : 60 %
Spectre ¹H RMN dans CDCl₃ à 200 MHz, δ en ppm
δ = 7,84 (d, 4H, J = 8,5 Hz, système AB OTS)
δ = 7,37 (d, 4H, J = 8,5 Hz, système AB OTS)
δ = 6,89 à 6,78 (m, 12H, Ar)
δ = 4,22 à 4,18 (m, 4H, OCH₂CH₂)
δ = 3, 89 à 3,82 (m, 4H, OCH₂CH₂)
δ = 2,39 (s, 6H, ArCH₃)

### e) Préparation du calixarène-bis-couronne 17

Dans un ballon de 250 ml, on introduit 0,425 g (1 mmol) de calix[4]arène **5**, 1,380 g (10 mmol) de K₂CO₃ dans 100 ml de CH₃CN. On agite à température ambiante pendant 4 heures puis on ajoute 0,691 g (1mmol) de ditosylate **14** et on chauffe sous reflux pendant 7 jours. La même quantité de K₂CO₃ 1,38 g (10 mmol) et de ditosylate 0,691 g (1 mmol) est encore une fois ajoutée à la solution. Le mélange réactionnel est remis sous reflux pendant 7 jours supplémentaire. Après 14 jours de reflux, le solvant est évaporé à sec. Le résidu obtenu est solubilisé dans le dichlorométhane et K₂CO₃ neutralisé avec HCl 1N. La phase organique est séchée sur sulfate de sodium, puis concentrée. Après purification par chromatographie sur colonne (CH₂Cl₂ : acétone - 90 : 10) le produit tribenzo-bis-couronne **17** est récupéré sous forme d'un solide blanc (340 mg).
Rendement : 30 %.
Spectre ¹H RMN dans CDCl₃ à 200 MHz, δ en ppm
δ = 7,22 à 6, 94 (m, 24H, Ar)
δ = 6,69 (d, 8H, J = 7 Hz, H méta du Ar)
δ = 6,55 (t, 4H, J = 7 Hz, H para du Ar)
δ = 3,72 (s, 8H, Ar-CH₂Ar)
δ = 3,21 à 3,17 (m, 16H, OCH₂CH₂O)

Les exemples qui suivent illustrent l'utilisation des calixarènes de l'invention pour l'extraction sélective du césium

### Exemple 5 : Extraction du césium

Dans cet exemple, on extrait le césium présent dans une solution aqueuse ayant une concentration en acide nitrique de 1 mol/l, contenant 5.10⁻⁴ mol/l de CsNO₃, au moyen des calixarènes de l'invention.

Dans ce but, on met un volume de solution aqueuse en contact avec un volume de liquide organique constitué par de l'ortho-nitrophénylhexyl éther contenant 10⁻² mol/l de calixarène. Lorsque l'équilibre est atteint, on mesure par spectrométrie gamma, la teneur en césium du liquide organique. On détermine ensuite le pourcentage de césium extrait et le coefficient de distribution D_{Cs} qui correspond au rapport de la concentration en césium dans le liquide organique à la concentration en césium de la solution aqueuse à l'équilibre.

Pour ces expériences, on utilise les caiixarénes des exemples 1 et 3 ci-dessus. Les résultats obtenus sont donnés dans le tableau 1.

A titre comparatif, on a donné également dans le tableau 1 les résultats obtenus dans les mêmes conditions avec des calixarènes de l'art antérieur, qui sont les calixarènes bis-couronnes des exemples 3 et 6b du document WO94/12502 et le calixarène bis-1,2-naphto couronne-6 et les calixarènes monocouronnes des exemples 3, 4 et 6 du document WO94/24138.

### Exemple 6 : Extraction du sodium

Dans cet exemple, on part d'une solution aqueuse ayant une concentration en acide nitrique de 1 mol/l contenant 5.10⁻⁴ mol/l de nitrate de sodium, et on la soumet à une extraction dans les mêmes conditions que celles de l'exemple 5 en utilisant un calixarène à une concentration de 10⁻² mol/l dans de l'orthonitrophényl hexyl éther. On mesure comme précédemment la teneur en sodium de la solution organique par spectrométrie gamma et on évalue le pourcentage de sodium extrait et le coefficient de distribution D_{Na}.
On emploie les mêmes calixarènes que dans l'exemple 5. Les résultats obtenus sont donnés dans le tableau 1.

### Exemple 7 : Extraction du césium

On suit le même mode opératoire que dans l'exemple 5 pour extraire le césium à partir d'une solution aqueuse de composition différente de celle de l'exemple 1, qui contient :
1 mol/l d'acide nitrique,
4 mol/l de nitrate de sodium, et
5.10⁻⁴ mol/l de CsNO₃.

Les calixarènes et le solvant utilisé sont les mêmes que ceux de l'exemple 1.

Les résultats obtenus sont donnés dans le tableau 1.

Les résultats du tableau 1 montrent que les caiix[4]arènes monocouronnes et bis-couronnes de l'invention extraient le césium avec de bons rendements et le séparent du sodium présent également dans les effluents issus des usines de retraitement. Par ailleurs, ils montrent qu'en présence de fortes concentrations de sodium, les calixarènes de l'invention permettent l'extraction sélective de quantités beaucoup plus importantes de césium alors que l'inverse se produit avec les calixarènes de l'art antérieur.

En effet, D_{Cs} augmente de 31 à 56 ou de 23 à 54 avec les calixarènes de l'invention alors que D_{Cs} diminue dans tous les cas avec les calixarènes de l'art antérieur.

On obtient donc avec les calixarènes de l'invention un effet surprenant sur l'extraction du césium.

### Exemple 8 : Transport du césium.

Dans cet exemple, on effectue l'extraction du césium-137 en présence de sodium-22 à partir d'une solution contenant 1 mol/l d'acide nitrique, 4 mol/l de NaNO₃, du ¹³⁷Cs et du ²²Na avec une activité en césium 137 de 1558 kBq/l et une activité en sodium-22 de 505 kBq/l.

Pour réaliser l'extraction, on utilise un dispositif séparé en deux compartiment par une membrane licuide supportée, constituée par une membrane microporeuse en polypropylène commercialisée sous le nom CELGARD 2500 qui présente les caractéristiques suivantes :
Facteur de porosité : 0,45.
Diamètre des pores : 0,04 µm.
Epaisseur de la membrane : 0,025 mm.

Les pores de cette membrane sont remplis d'une phase organique constituée de nitrophénylhexyl éther comprenant 0,01 mol/l du calixarène de l'exemple 3.

Les deux compartiments du dispositif sont munis chacun d'un agitateur magnétique. Le premier compartiment contient 55 ml de la solution aqueuse de départ et le second compartiment contient une solution aqueuse de réextraction constituée par de l'eau. La surface efficace de la membrane est de 11,6 cm².

Pendant cet essai, on détermine l'activité de la solution aqueuse de départ du premier compartiment par spectrométrie γ et on en déduit le pourcentage de césium 137 extrait dans le second compartiment.

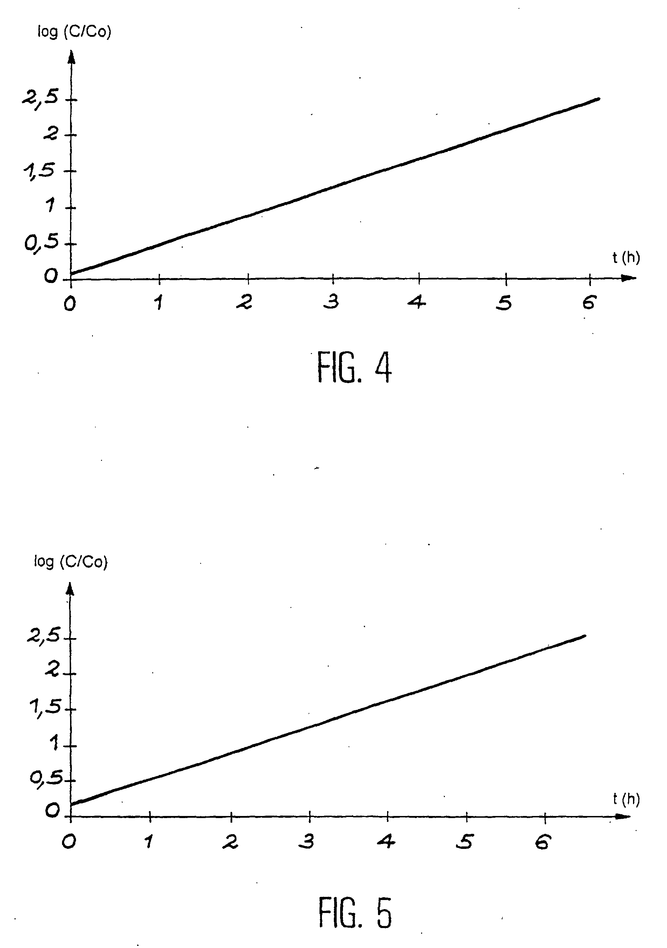

Les résultats obtenus sont donnés dans le tableau 2 et sur la figure 4.

Cette figure 4 représente l'évolution de la concentration de césium dans la solution de réextraction en fonction du temps (en heures).

La concentration en césium est donnée en ordonnées par log (C/Co) avec C représentant la concentration en césium au temps t et Co la concentration initiale.

Le transport correspond à une perméabilité de la membrane au césium 137 de 4,27 cm/h, celui du sodium n'est pas mesurable. Perméabilité inférieure à 0,1 cm/h.

### Exemple 9 : Extraction du césium 137.

Dans cet exemple, on suit le même mode opératoire que dans l'exemple 8 en utilisant une solution aqueuse de départ contenant 1 mol/l de HNO₃, 4 mol/l de NaNO₃, du ¹³⁷Cs et du ²²Na avec une activité en césium 137 de 1549 kBq/l et une activité en sodium-22 de 505 kBq/l. Dans ce cas, les pores de la membrane sont remplis d'une phase organique constituée de nitrophénylhexyl éther comprenant 0,01 mol/l du calixaréne-monocouronne de i'exemple 1. La solution de reextraction est de l'eau comme dans l'exemple 8.

Les résultats obtenus sont donnés dans le tableau 3 et sur la figure 5.

La figure 5 représente l'évolution de la concentration en césium (en log C/Co) de la solution de réextraction en fonction du temps (en heures).

Dans ces conditions, la perméabilité de la membrane vis-à-vis du césium 137 est de 4,19 cm/h, celle du sodium inférieure à 0,1 cm/h.

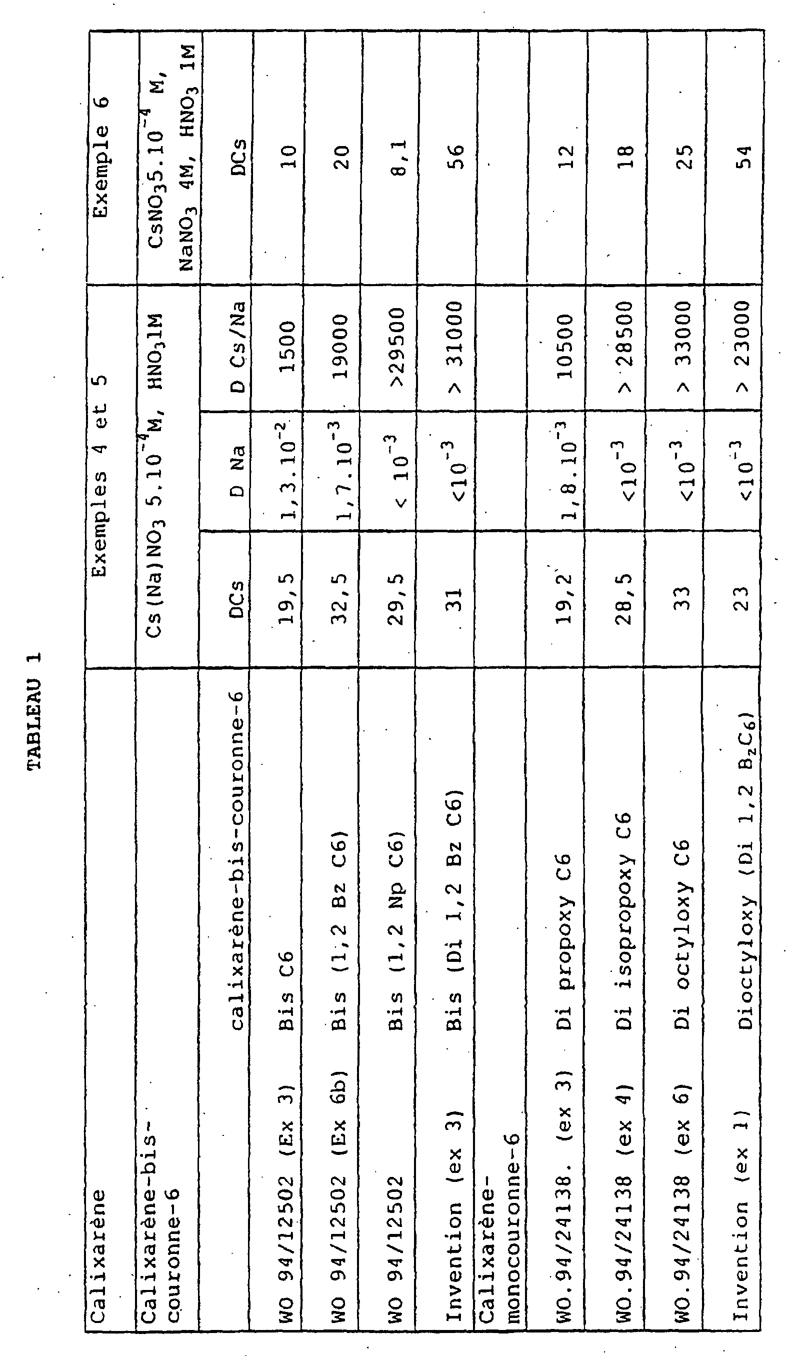

**Tableau 2**

| Temps (heures) | Activité en ¹³⁷CS de la solution aqueuse de départ (kBq/l) | ¹³⁷Cs extrait (en %) |
|---|---|---|
| 0 | 1558 | 0,0 % |
| 0,5 | 1192 | 23,5 % |
| 1 | 1057 | 32,2 % |
| 1,5 | 827 | 46,9 % |
| 2 | 685 | 56,0 % |
| 2,5 | 569 | 63,5 % |
| 3 | 481 | 69,1 % |
| 3,5 | 367 | 76,4 % |
| 4 | 297 | 80,9 % |
| 4,5 | 242 | 84,5% |
| 5 | 208 | 86,6 % |
| 5,5 | 180 | 88,4 % |
| 6 | 134 | 91,4 % |

**TABLEAU 3**

| Temps (heures) | Activité en ¹³⁷Cs de la solution aqueuse de départ (kBq/l) | ¹³⁷Cs extrait (en %) |
|---|---|---|
| 0 | 1549 | 0,0 % |
| 0,5 | 1173 | 24,3 % |
| 1 | 935 | 39,6 % |
| 1,5 | 723 | 53,3 % |
| 2 | 567 | 63,4 % |
| 2,5 | 444 | 71,3 % |
| 3,5 | 339 | 78,1 % |
| 4,5 | 243 | 84,3 % |
| 5 | 207 | 86,6 % |
| 5,5 | 180 | 88,4 % |
| 6 | 158 | 89,8 % |
| 6,5 | 146 | 90,6 % |

## Revendications

1. Calix[4]arène-couronne répondant à la formule : 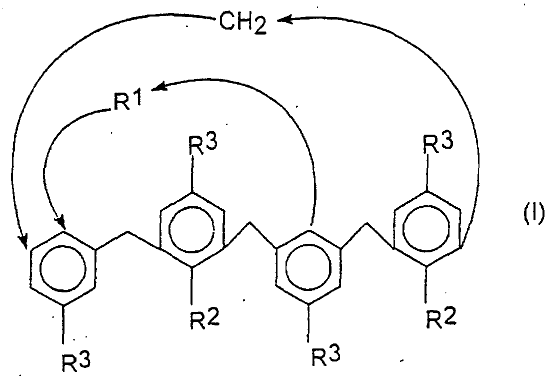 dans laquelle :
- R¹ représente un groupe de formule :
O-(CH₂CH₂O)ₘ- (XO)ₙ- (CH₂CH₂O)ₚ-(YO)ᵣ-(CH₂CH₂O)ₛ (II),
où X et Y qui peuvent être identiques ou différents, représentent un groupe arylène ou cycloalkylène, m, n, p, r et s sont des nombres entiers tels que :
m = s = 1,
1 ≤ n ≤ 3,
p = 0 ou 1,
r = 0 ou 1,
- R² représente le groupe OH ou un groupe alcoxy de 1 à 18 atomes de carbone, les deux R² pouvant être identiques ou différents, ou les deux R² forment ensemble un groupe de formule :
O-(CH₂CH₂O)ₘ-(XO)ₙ-(CH₂CH₂O)ₚ-(YO)ᵣ-(CH₂CH₂O)ₛ (II),
dans laquelle X, Y, m, n, p, r et s sont tels que définis ci-dessus ; et
- R³ représente un atome d'hydrogène ou un groupe alkyle de 1 à 3 atomes de carbone,
à condition que, dans le cas où les deux R² forment ensemble un groupe de formule (II), p et r ne soient pas tous deux égaux à 0 lorsque n = 1.

2. Calix[4]arène-couronne selon la revendication 1 répondant à la formule (I) avec R² représentant le goupe OH ou un groupe alcoxy.

3. Calix[4]arène-couronne selon la revendication 1 répondant à la formule (I) dans laquelle les deux R² forment ensemble un groupe identique à R¹.

4. Calix[4]arène-couronne selon l'une quelconque des revendications 1 à 3 dans lequel R1 représente le groupe de formule (III) ou (IV) : 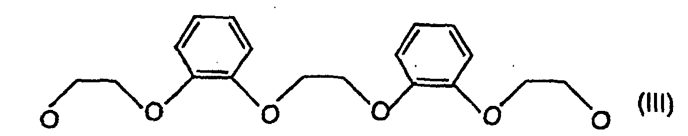 ou

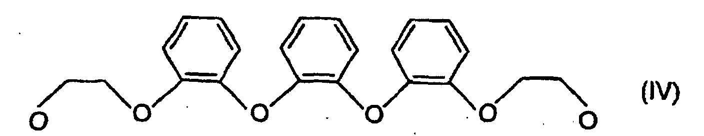

5. Calix[4]arène-couronne selon la revendication 2 dans lequel R¹ représente le groupe de formule : 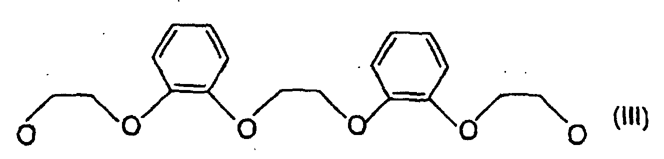 R² représente le groupe octoxy, et R³ représente un atome d'hydrogène.

6. Calix[4]arène-couronne selon la revendication 2, dans lequel R¹ représente le groupe de formule 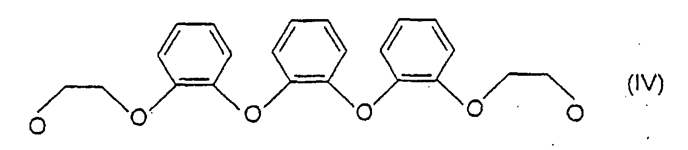 R² représente le groupe octoxy, et
R³ représente un atome d'hydrogène.

7. Calix[4]arène-couronne selon la revendication 3, dans lequel R¹ représente le groupe de 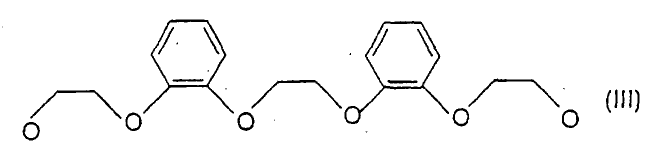 et R³ représente un atome d'hydrogène.

8. Calix[4]arène-couronne selon la revendication 3, dans lequel R¹ représente le groupe de formule : 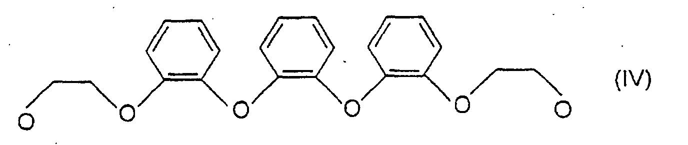 et R³ représente un atome d'hydrogène.

9. Procédé de préparation d'un calix[4]arène-couronne répondant à la formule : 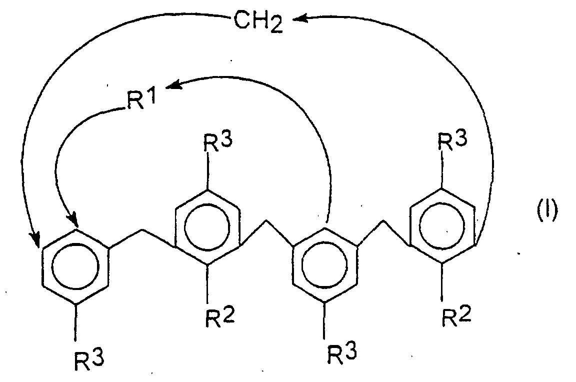 dans laquelle :
- R¹ représente un groupe de formule :
O-(CH₂CH₂O)ₘ-(XO)ₙ-(CH₂CH₂O)ₚ-(YO)ᵣ-(CH₂CH₂O)ₛ (II),
où X et Y qui peuvent être identiques ou différents, représentent un groupe arylène ou cycloalkylène, m, n, p, r et s sont des nombres entiers tels que :
m = s = 1,
1 ≤ n ≤ 3,
p = 0 ou 1,
r = 0 ou 1,
- R² représente le groupe OH ou un groupe alcoxy de 1 à 18 atomes de carbone, et
- R³ représente un atome d'hydrogène ou un groupe alkyle de 1 à 3 atomes de carbone,
**caractérisé en ce qu'**il consiste à faire réagir une calix[4]arène de formule : 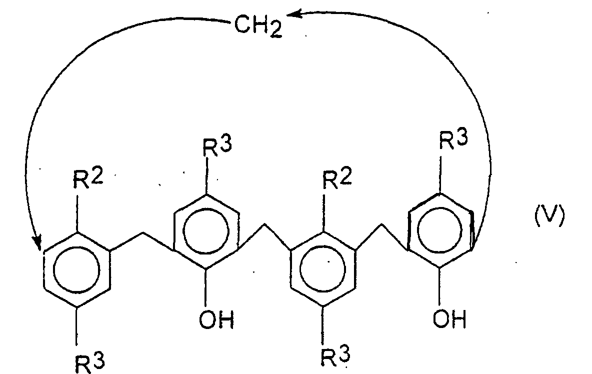 dans laquelle R² et R³ ont les significations données ci-dessus avec un ditosylate de formule (VI) : 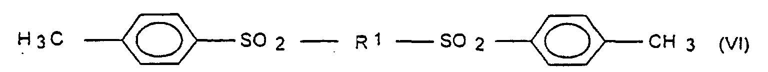 dans laquelle R¹ a la signification donnée ci-dessus.

10. Procédé de préparation d'un calix[4]arène répondant à la formule : 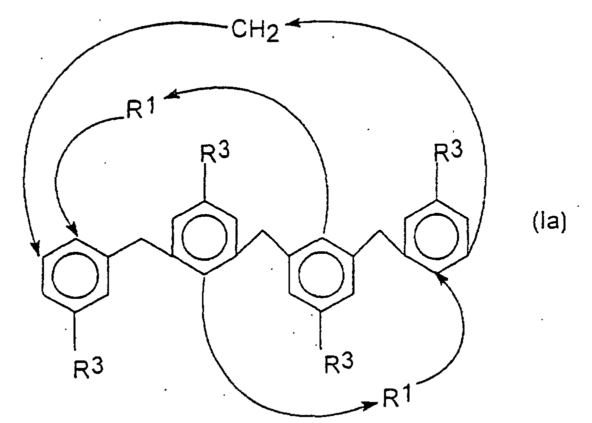 dans laquelle :
- R¹ représente un groupe de formule :
O-(CH₂CH₂O)ₘ- (XO)ₙ- (CH₂CH₂O)ₚ-(YO)ᵣ- (CH₂CH₂O)ₛ, (II),
où X et Y qui peuvent être identiques ou différents, représentent un groupe arylène ou cycloalklène, m, n, p, r, et s sont des nombres entiers tels que :
m = s = 1,
1 ≤ n ≤ 3,
p = 0 ou 1,
r = 0 ou 1,
- R³ représente un atome d'hydrogène ou un groupe alkyle de 1 à 3 atomes de carbone,
**caractérisé en ce qu'**il consiste à faire réagir le calix[4]arène de formule : 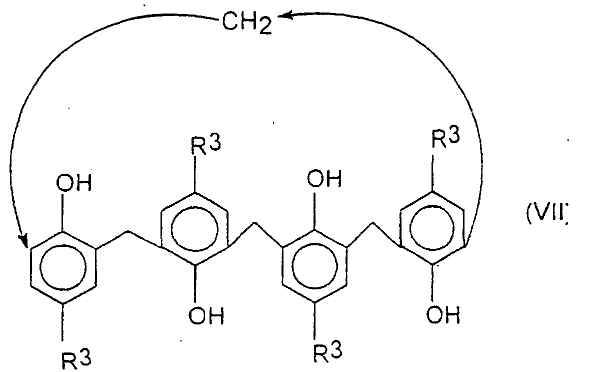 avec un ditosylate de formule : 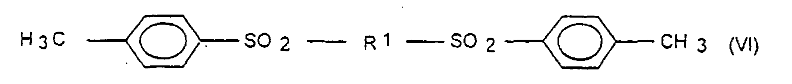 dans laquelle R¹ a la signification donnée ci-dessus.

11. Procédé selon la revendication 9 ou 10 dans lequel R¹ étant le groupe de formule : 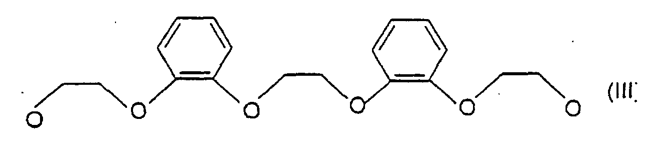 on prépare le tosylate en effectuant les étapes suivantes :
a) synthèse du 2-(2-hydroxyéthoxy)phénol de formule : 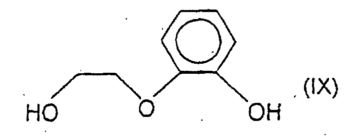 par réaction du catéchol avec du carbonate d'éthylène,
b) réaction du 2-(2-hydroxyéthoxy) phénol obtenu en a) avec du ditosylate d'éthylène glycol pour obtenir le 1,2-bis[2-(2-hydroxyéthoxy)phénoxyléthane, et
c) préparation du tosylate par réaction du 1,2-bis[2-(2-hydroxyéthoxy)phénoxy]éthane avec du chlorure de tosyle de formule :

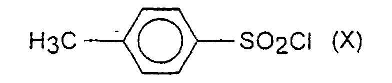

12. Procédé selon la revendication 9 ou 10 dans lequel R¹ étant le groupe de formule : 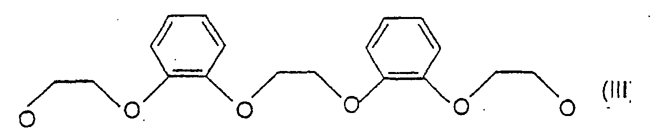 on prépare le tosylate en effectuant les étapes suivantes :
a) réaction du catéchol avec du 2-chloroéthanol pour obtenir le 2-(2-hydroxyéthcxy)phénol de formule : 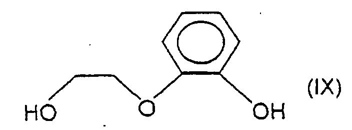
b) réaction du 2-(2-hydroxyéthoxy)phénol obtenu en a) avec du 1,2dibromoéthane pour obtenir le 1,2-bis[2-(2-hydroxyéthoxy)phénoxy]éthane, et
c) préparation du tosylate par réaction du 1,2-bis[2-(2-hydroxyéthoxy)phénoxy]éthane avec du chlorure de tosyle de formule (X) :

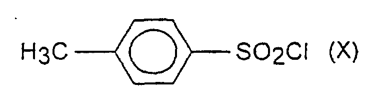

13. Procédé selon la revendication 9 ou 10 dans lequel R¹ représentant le groupe de formule : 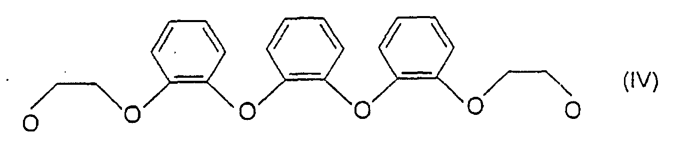 on prépare le tosylate en effectuant les étapes suivantes :
a) préparation du diphénol de formule : 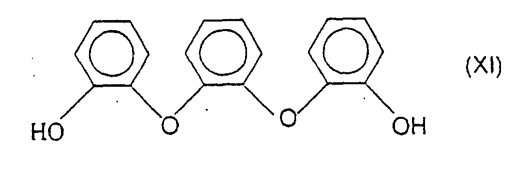
b) réaction du diphénol avec du ditosylate d'éthylène glycol.

14. Procédé selon la revendication 9 ou 10, dans lequel R¹ représentant le groupe de formule : 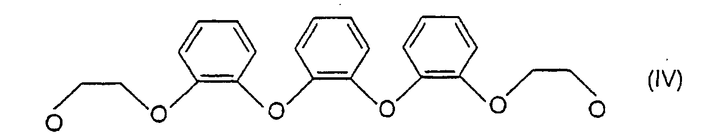 on prépare le tosylate en effectuant les étapes suivantes :
a) préparation du diphénol de formule : 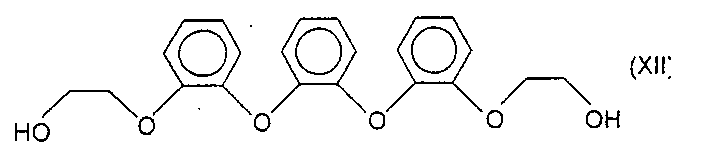
b) réaction du diphénol avec du chlorure de tosyle

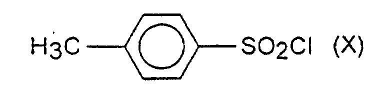

15. Procédé d'extraction sélective du césium à partir d'une solution aqueuse, qui consiste à mettre en contact cette solution aqueuse avec une phase liquide immiscible comprenant une calix[4]arène -couronne selon l'une quelconque des revendications 1 à 8, et à séparer de la solution aqueuse la phase immiscible ayant extrait le césium.

16. Procédé selon la revendication 14, dans lequel la phase liquide immiscible est une solution du calix[4]arène-couronne dans un solvant organique.

17. Procédé selon la revendication 15, dans lequel le solvant organique est un nitrophényl alkyl éther.

18. Procédé selon la revendication 14 dans lequel on récupère ensuite le césium dans une solution aqueuse de réextraction par mise en contact de la phase liquide ayant extrait le césium avec une solution aqueuse.

19. Procédé selon la revendication 17, dans lequel la solution aqueuse de réextraction est de l'eau distillé et désionisée.

20. Procédé selon l'une quelconque des revendications 17 et 18, dans lequel la phase liquide immiscible forme une membrane liquide, on met en contact la solution aqueuse contenant le césium avec une surface de cette membrane et on met en contact la solution aqueuse de réextraction avec la surface opposée de cette membrane liquide.

21. Procédé selon l'une quelconque des revendiations 14 à 19, dans lequel la solution aqueuse de départ est un effluent aqueux contenant du césium, avec ou sans sodium provenant d'une installation de retraitement de combustibles nucléaires usés.

## Patentansprüche

1. Krone-calix[4]aren entsprechend der Formel: 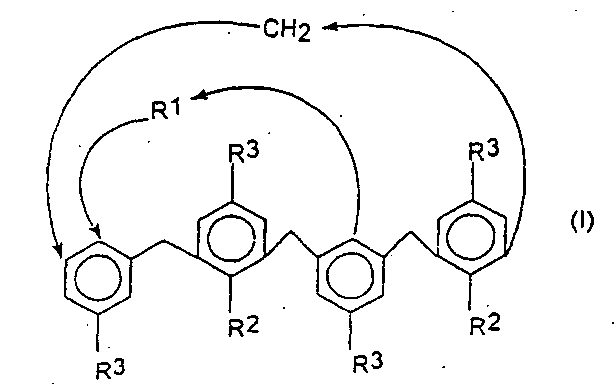 in welcher:
- R¹ eine Gruppe der Formel:
O-(CH₂CH₂O)ₘ-(XO)ₙ-(CH₂CH₂O)ₚ-(YO)ᵣ-(CH₂CH₂O)ₛ (II)
darstellt, wo X und Y, die identisch oder verschieden sein können, eine Arylen- oder Cycloalkylengruppe darstellen, m, n, p, r und s solche ganzen Zahlen sind, dass:
m = s = 1,
1 ≤ n ≤ 3,
p = 0 oder 1,
r = 0 oder 1,
- R² die OH-Gruppe oder eine Alkoxygruppe von 1 bis 18 Kohlenstoffatomen darstellt, wobei die beiden R² identisch oder verschieden sein können oder die beiden R² zusammen eine Gruppe der Formel:
O-(CH₂CH₂O)ₘ-(XO)ₙ-(CH₂CH₂O)ₚ-(YO)ᵣ-(CH₂CH₂O)ₛ (II)
bilden, in welcher X, Y, m, n, p, r und s wie oben definiert sind, und
- R³ ein Wasserstoffatom oder eine Alkylgruppe von 1 bis 3 Kohlenstoffatomen darstellt,
unter der Bedingung, dass im Fall, wo die beiden R² zusammen eine Gruppe der Formel (II) bilden, p und r nicht beide gleich 0 sind, wenn n = 1.

2. Krone-calix[4]aren nach Anspruch 1 entsprechend Formel (I) mit R², das die OH-Gruppe oder eine Alkoxygruppe darstellt.

3. Krone-calix[4]aren nach Anspruch 1 entsprechend Formel (I); in welcher die beiden R² zusammen eine mit R² identische Gruppe bilden.

4. Krone-calix[4]aren nach einem der Ansprüche 1 bis 3, in welchem R¹ die Gruppe der Formel: 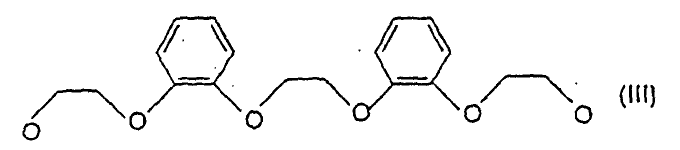 oder 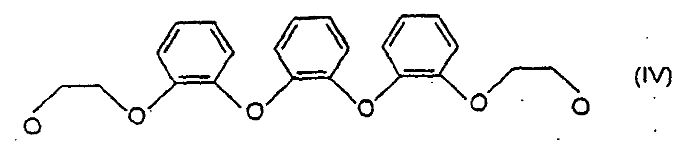 darstellt.

5. Krone-calix[4]aren nach Anspruch 2, in welchem R¹ die Gruppe der Formel: 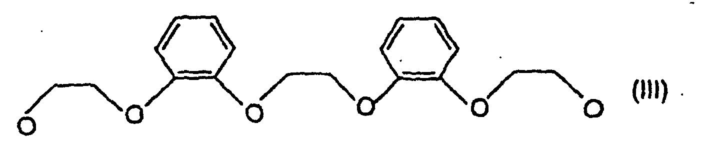 darstellt, R² die Octoxygruppe darstellt und R³ ein Wasserstoffatom darstellt.

6. Krone-calix[4]aren nach Anspruch 2, in welchem R¹ die Gruppe der Formel: 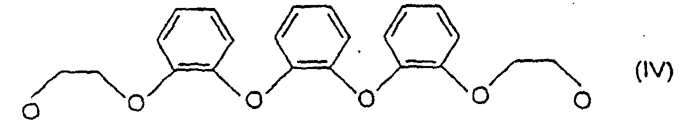 darstellt, R² die Octoxygruppe darstellt und R³ ein Wasserstoffatom darstellt.

7. Krone-calix[4]aren nach Anspruch 3, in welchem R¹ die Gruppe der Formel: 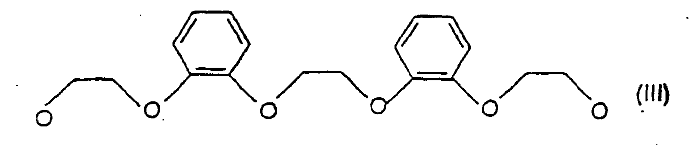 darstellt und R³ ein Wasserstoffatom darstellt.

8. Krone-calix[4]aren nach Anspruch 3, in welchem R¹ die Gruppe der Formel: 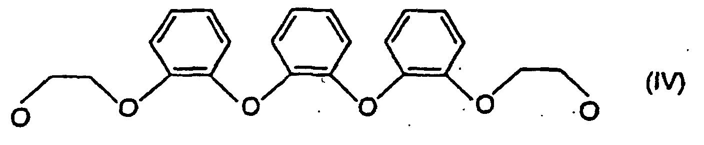 darstellt und R³ ein Wasserstoffatom darstellt.

9. Verfahren zur Herstellung eines Krone-calix[4]arens entsprechend der Formel: 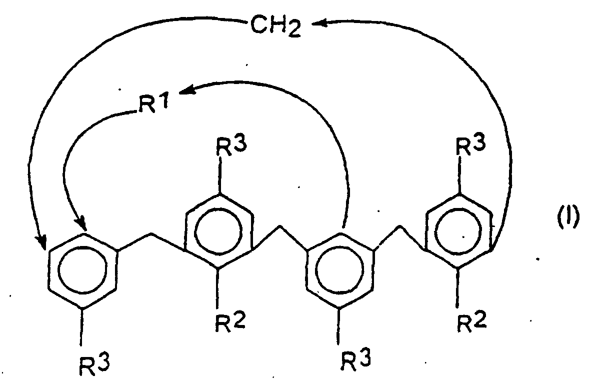 in welcher:
- R¹ eine Gruppe der Formel:
O-(CH₂CH₂O)ₘ-(XO)ₙ-(CH₂CH₂O)ₚ-(YO)ᵣ-(CH₂CH₂O)ₛ (II)
darstellt, wo X und Y, die identisch oder verschieden sein können, eine Arylen- oder Cycloalkylengruppe darstellen, m, n, p, r und s solche ganzen Zahlen sind, dass:
m = s = 1,
1 ≤n≤3,
p = 0 oder 1,
r = 0 oder 1,
- R² die OH-Gruppe oder eine Alkoxygruppe von 1 bis 18 Kohlenstoffatomen darstellt und
- R³ ein Wasserstoffatom oder eine Alkylgruppe von 1 bis 3 Kohienstoffatomen darstellt,
**dadurch gekennzeichnet, dass** es darin besteht, ein Calix[4]aren der Formel: 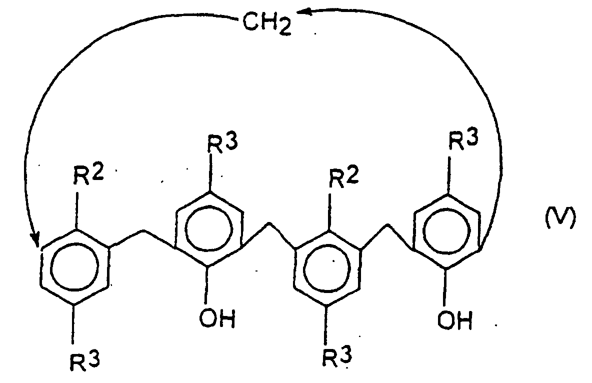 in welchem R² und R³ die oben gegebenen Bedeutungen haben, mit einem Ditosylat der Formel: 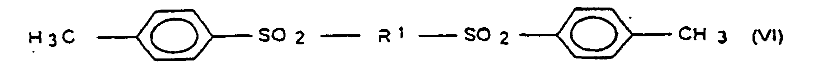 in welchem R¹ die oben gegebene Bedeutung hat, reagieren zu lassen.

10. Verfahren zur Herstellung eines Krone-calix[4]arens entsprechend der Formel: 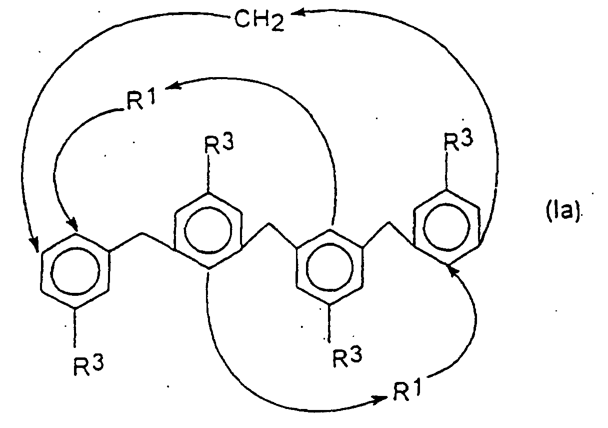 in welcher:
- R¹ eine Gruppe der Formel:
O-(CH₂CH₂O)ₘ-(XO)ₙ-(CH₂CH₂O)ₚ-(YO)ᵣ-(CH₂CH₂O)ₛ (II)
darstellt, wo X und Y, die identisch oder verschieden sein können, eine Arylen- oder Cycloalkylengruppe darstellen, m, n, p, r und s solche ganzen Zahlen sind, dass:
m = s = 1,
1 ≤n≤3,
p = 0 oder 1,
r = 0 oder 1,
- R³ ein Wasserstoffatom oder eine Alkylgruppe von 1 bis 3 Kohlenstoffatomen darstellt,
**dadurch gekennzeichnet, dass** es darin besteht, ein Calix[4]aren der Formel: 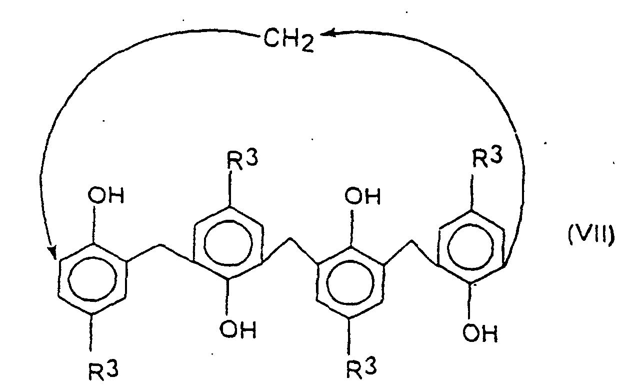 mit einem Ditosylat der Formel: 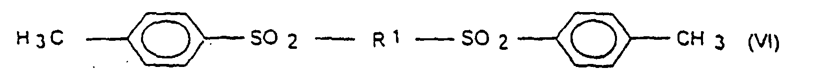 in welchem R¹ die oben gegebene Bedeutung hat, reagieren zu lassen.

11. Verfahren nach Anspruch 9 oder 10, in welchem R¹ die Gruppe der Formel 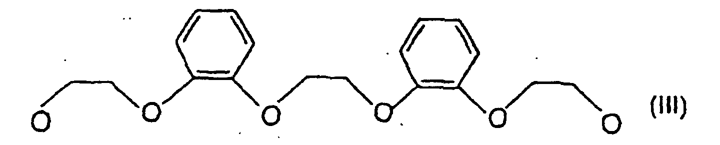 ist. Man stellt das Tosylat her, indem man die folgenden Schritte ausführt:
a) Synthese des 2-(2-Hydroxyethoxy)phenols der Formel: 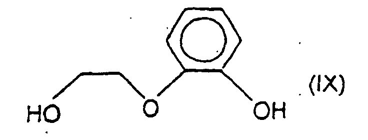 durch Reaktion von Brenzcatechin mit Ethylencarbonat,
b) Reaktion des in a) erhaltenen 2-(2-Hydroxyethoxy)phenols mit dem Ditosylat des Ethylenglykols um das 1,2-Bis[2-(2-hydroxyethoxy)phenoxy]ethan zu erhalten, und
c) Herstellung des Tosylats durch Reaktion des 1,2-Bis[2-(2-hydroxy-ethoxy)phenoxy]ethans mit Tosylchlorid der Formel:

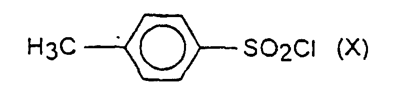

12. Verfahren nach Anspruch 9 oder 10, in welchem R¹ die Gruppe der Formel: 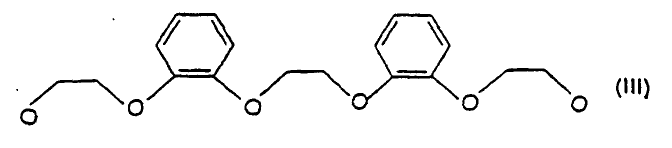 ist. Man stellt das Tosylat her, indem man die folgenden Schritte ausführt:
a) Reaktion von Brenzcatechin mit 2-Chlorethanol um das 2-(2-Hydroxyethoxy)phenol der Formel: 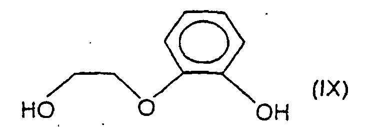 zu erhalten,
b) Reaktion des in a) erhaltenen 2-(2-Hydroxyethoxy)phenols mit 1,2-Dibromethan um das 1,2-Bis[2-(2-hydroxyethoxy)phenoxy]ethan erhalten, und
c) Herstellung des Tosylats durch Reaktion des 1,2-Bis[2-(2-hydroxyethoxy)phenoxy]ethans mit Tosylchlorid der Formel:

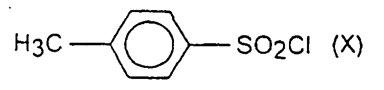

13. Verfahren nach Anspruch 9 oder 10, in welchem R¹ die Gruppe der Formel: 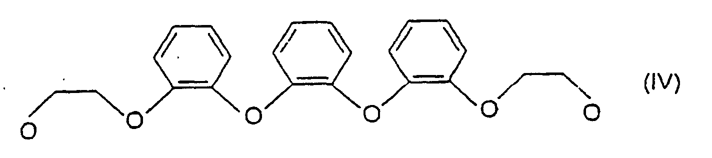 darstellt. Man stellt das Tosylat her, indem man die folgenden Schritte ausführt:
a) Herstellung des Diphenols der Formel: 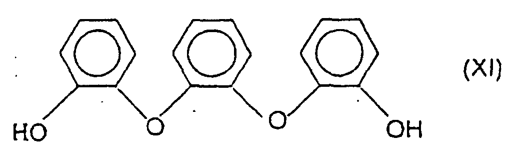
b) Reaktion des Diphenols mit dem Ditosylat des Ethylenglykols.

14. Verfahren nach Anspruch 9 oder 10, in welchem R¹ die Gruppe der Formel: 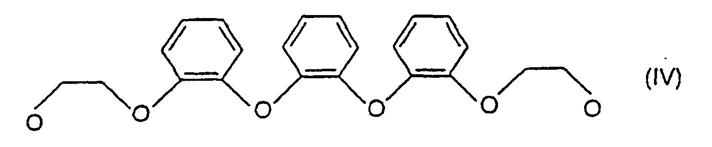 darstellt. Man stellt das Tosylat her, indem man die folgenden Schritte ausführt:
a) Herstellung des Diphenols der Formel: 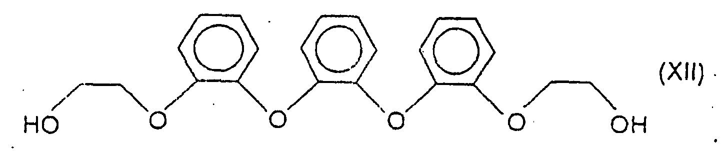
b) Reaktion des Diphenols mit Tosylchlorid:

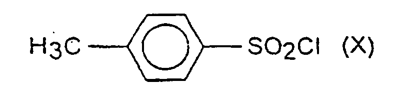

15. Verfahren zur selektiven Extraktion des Caesiums aus einer wässrigen Lösung, welches darin besteht, diese wässrige Lösung mit einer damit unmischbaren flüssigen, ein Krone-calix[4]aren nach einem der Ansprüche 1 bis 8 enthaltenden Phase in Kontakt zu bringen und von der wässrigen Lösung, die unmischbare Phase, die das Caesium extrahiert hat, abzutrennen.

16. Verfahren nach Anspruch 14, in welchem die unmischbare flüssigePhase eine Lösung des Krone-calix[4]arens in einem organischen Lösungsmittel ist.

17. Verfahren nach Anspruch 15, in welchem das organische Lösungsmittel ein Nitrophenylalkylether ist.

18. Verfahren nach Anspruch 14, in welchem man anschließend durch In-Kontakt-Bringen der flüssigen Phase, die das Caesium extrahiert hat, mit einer wässrigen Lösung das Caesium in einer wässrigen Reextraktionslösung zurückgewinnt.

19. Verfahren nach Anspruch 17, in welchem die wässrige Reextraktionslösung destilliertes und deionisiertes Wasser ist.

20. Verfahren nach einem der Ansprüche 17 und 18, in welchem die unmischbare flüssige Phase eine flüssige Membran bildet, man die wässrige Lösung, die das Caesium enthält, mit einer Oberfläche dieser Membran in Kontakt bringt und die wässrige Reextraktionslösung mit der entgegengesetzten Oberfläche dieser flüssigen Membran in Kontakt bringt.

21. Verfahren nach einem der Ansprüche 14 bis 19, in welchem die wässrige Ausgangslösung ein wässriges, Caesium mit oder ohne Natrium enthaltendes Abwasser ist, das von einer Anlage zur Wiederaufarbeitung verbrauchter Kernbrennstoffe stammt.

## Claims

1. A crown calix[4]arene corresponding to the formula : 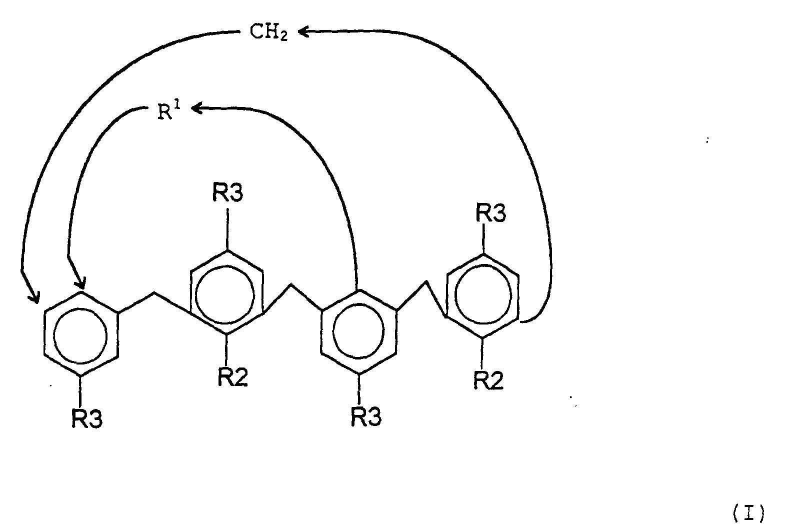 in which :
- R¹ represents a group of formula :
O-(CH₂CH₂O)ₘ- (XO)ₙ- (CH₂CH₂O)ₚ- (YO)ᵣ-(CH₂CH₂O)ₛ (II),
where X and Y, which can be identical or different, represent an arylene or cycloalkylene group, m, n, p, r and s are whole numbers such that :
m = s = 1,
1 ≤ n ≤ 3,
p = 0 or 1
r = 0 or 1,
- R² represents an OH group or an alkoxy group with from 1 to 18 carbon atoms, the two R² groups can be identical or different, or the two R² groups together form a group of formula :
O-(CH₂CH₂O)ₘ- (XO)ₙ- (CH₂CH₂O)ₚ- (YO)ᵣ-(CH₂CH₂O)ₛ (II),
in which X, Y, m, n, p, r and s are as defined above ; and
- R³ represents a hydrogen atom or an alkyl group with 1 to 3 carbon atoms,
with the condition that, in the case where the two R² groups together form a group of formula (III), p and r will not both be equal to 0 when n = 1.

2. A crown calix[4]arene according to Claim 1 corresponding to formula (I) with R² representing a OH group or an alkoxy group.

3. A crown calix[4]arene according to Claim 1 corresponding to formula (I) in which the two R² groups together form a group identical to R¹.

4. A crown calix[4]arene according to any one of Claims 1 to 3 in which R¹ represents the group of formula (III) or (IV) :

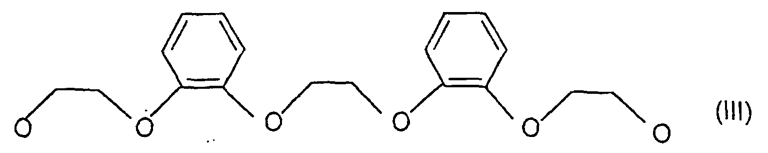

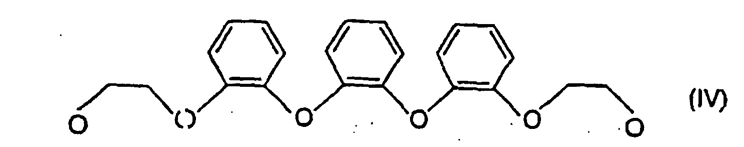

5. A crown calix[4]arene according to Claim 2 in which R¹ represents the group of formula : 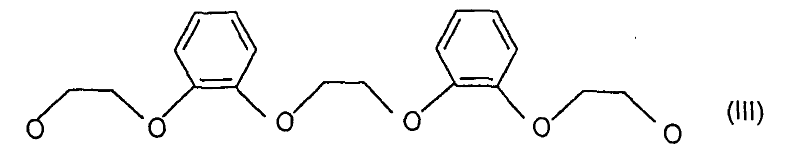 R² represents the octoxy group and R³ represents a hydrogen atom.

6. A crown calix[4]arene according to Claim 2 in which R¹ represents the group of formula : 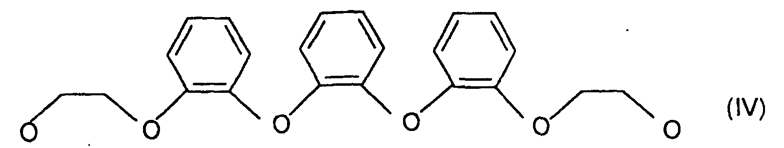 R² represents the octoxy group, and
R³ represents an atom of hydrogen.

7. A crown calix[4]arene according to Claim 3 in which R¹ represents the group of formula : 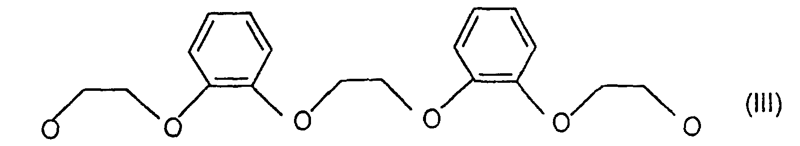 and R³ represents a hydrogen atom.

8. A crown calix[4]arene according to Claim 3 in which R¹ represents the group of formula : 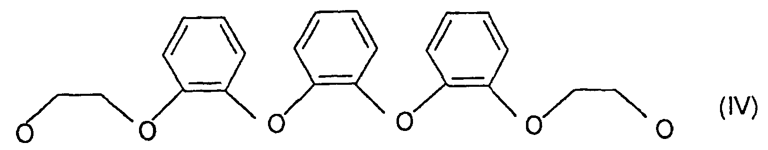 and R³ represents an atom of hydrogen.

9. A method of preparation of a crown calix[4]arene corresponding to the formula : 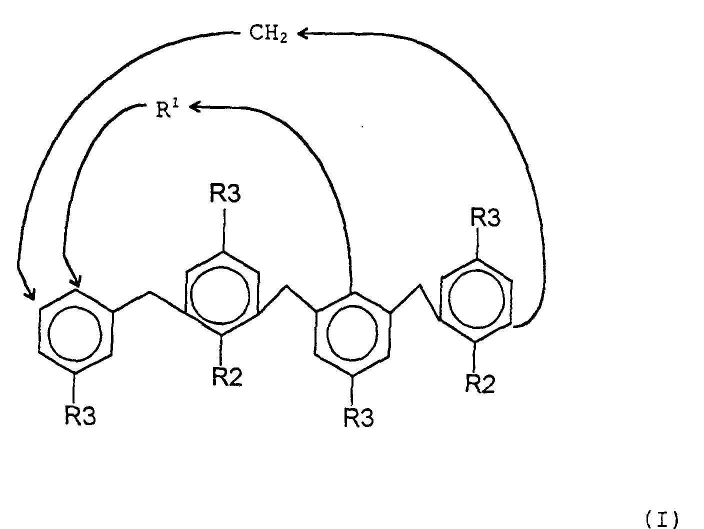 in which :
- R¹ represents a group of formula :
O- (CH₂CH₂O)ₘ- (XO)ₙ- (CH₂CH₂O)ₚ- (YO)ᵣ-(CH₂CH₂O)ₛ (II),
where X and Y, which can be identical or different, represent an arylene or cycloalkylene group, m, n, p, r and s are whole numbers such that :
m = s = 1,
1 ≤ n ≤ 3,
p = 0 or 1
r = 0 or 1,
- R² represents an OH group or an alkoxy group with from 1 to 18 carbon atoms, and
- R³ represents a hydrogen atom or an alkyl group with 1 to 3 carbon atoms,
**characterised in that** it consists of reacting a calix[4]arene of formula : 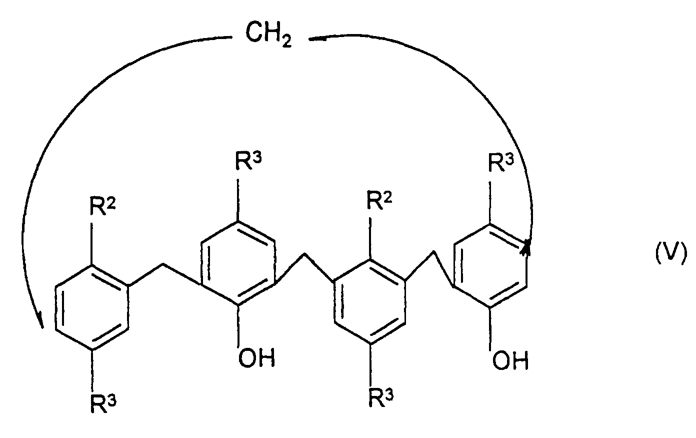 in which R² and R³ are as stated above, with a ditosylate of formula (VI) : 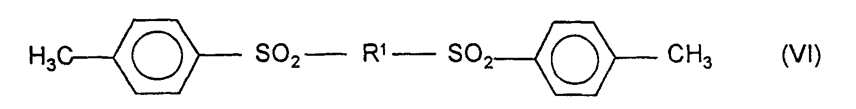 in which R¹ is as stated above.

10. A method of preparing a calix[4]arene corresponding to the formula : 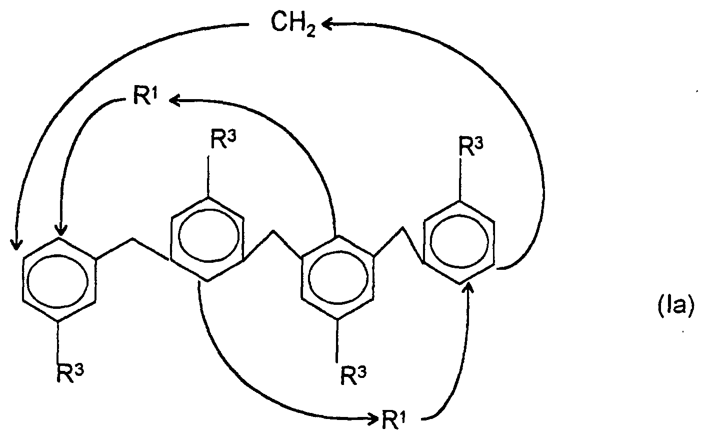 in which R¹ represents a group of formula :
O- (CH₂CH₂O)ₘ-(XO)ₙ- (CH₂CH₂O)ₚ- (YO)ᵣ-(CH₂CH₂O)ₛ (II),
where X and Y, which can be identical or different, represent an arylene or cycloalkylene group, m, n, p, r and s are whole numbers such that :
m = s = 1,
1 ≤ n ≤ 3,
p = 0 or 1
r = 0 or 1,
- R³ represents a hydrogen atom or an alkyl group with 1 to 3 carbon atoms,
**characterised in that** it consists of reacting a calix[4]arene of formula : 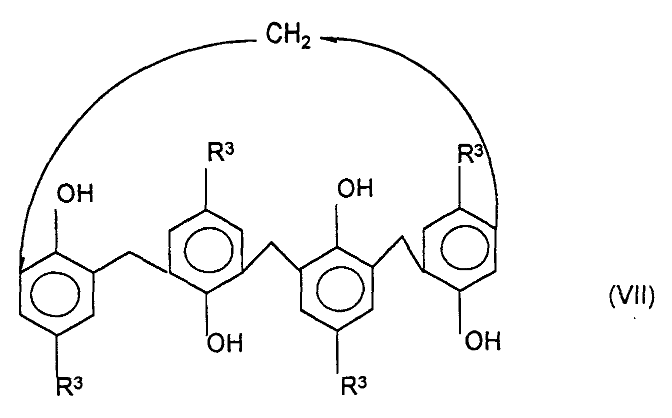 with a ditosylate of formula : 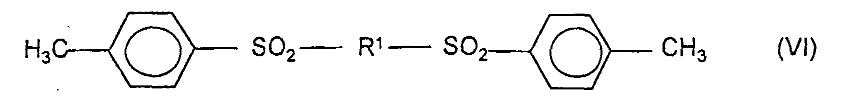 in which R¹ has the meaning stated above.

11. A method according to Claim 9 or 10 in which R¹ being the group of formula : 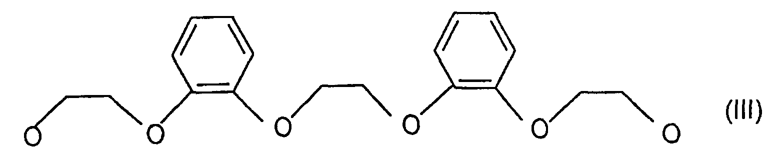 the tosylate is prepared by carrying out the following steps :
a) synthesis of 2-(2-hydroxyethoxy)phenol of formula : 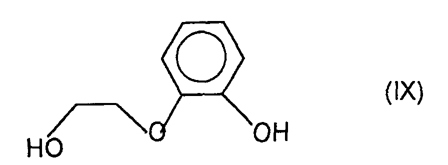 by reaction of catechol with ethylene carbonate
b) reaction of the 2-(2-hydroxyethoxy)phenol obtained in a) with ethylene glycol ditosylate to obtain 1,2-bis[2-(2-hydroxyethoxy)phenoxy]ethane and
c) preparation of the tosylate by reaction of 1,2-bis[2-(2-hydroxyethoxy)phenoxy]ethane with tosyl chloride of formula (X) :

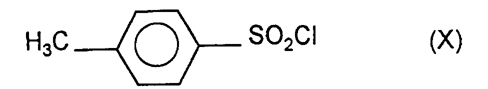

12. A method according to Claim 9 or 10 in which R¹ being the group of formula : 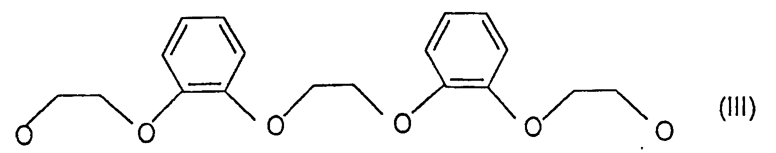 the tosylate is prepared by carrying out the following steps :
a) reaction of catechol with 2-chloroethanol to obtain 2-(2-hydroxyethoxy)phenol of formula : 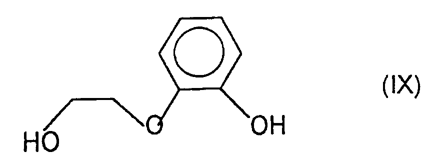
b) reaction of the 2-(2-hydroxyethoxy)phenol obtained in a) with 1,2-dibromoethane to obtain 1,2-bis[2-(2-hydroxyethoxy)phenoxy]ethane and
c) preparation of the tosylate by reaction of 1,2-bis[2-(2-hydroxyethoxy)phenoxy] ethane with tosyl chloride of formula (X) :

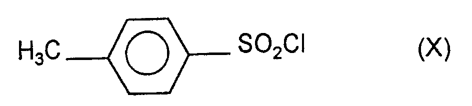

13. A method according to Claim 9 or 10 in which R¹ representing the group of formula : 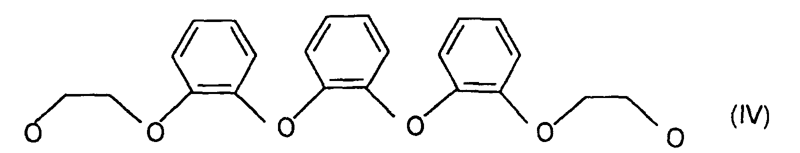 the tosylate is prepared by carrying out the following steps :
a) preparation of the diphenol of formula : 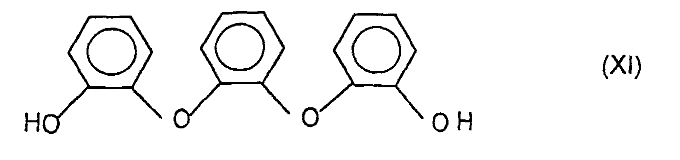
b) reaction of the diphenol with ethylene glycol ditosylate.

14. A method according to Claim 9 or 10 in which R¹ representing the group of formula : 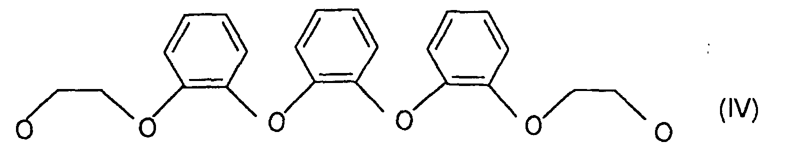 the tosylate is prepared by carrying out the following steps :
a) preparation of the diphenol of formula : 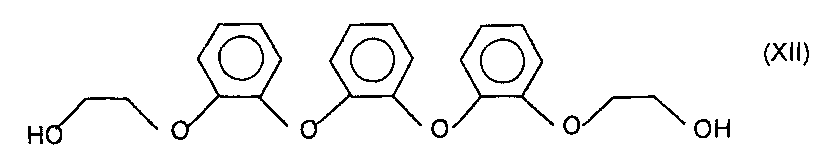
b) reaction of the diphenol with tosyl chloride :

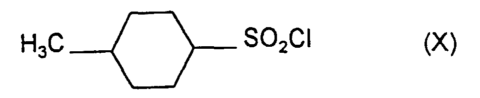

15. A method for the selective extraction of caesium from an aqueous solution that consists of bringing this solution into contact with an immiscible liquid phase that includes a crown calix[4]arene according to any one of Claims 1 to 8 and of separating the immiscible phase from the aqueous solution having extracted the caesium.

16. A method according to Claim 14, in which the immiscible liquid phase is a solution of the crown calix[4]arene in an organic solvent.

17. A method according to Claim 15, in which the organic solvent is a nitrophenyl alkyl ether.

18. A method according to Claim 14 in which afterwards, the caesium is recovered into an aqueous re-extraction solution by bringing the liquid phase which has extracted the caesium into contact with an aqueous solution.

19. A method according to Claim 17, in which the aqueous re-extraction solution is distilled an deionised water.

20. A method according to any one of Claims 17 and 18, in which the immiscible liquid phase forms a liquid membrane, the aqueous solution containing the caesium is brought into contact with one surface of this membrane and the aqueous re-extraction solution is brought into contact with the opposite surface of this liquid membrane.

21. A method according to any one of Claims 14 to 19, in which the aqueous starting solution is an aqueous effluent containing caesium with or without sodium arising from a spent nuclear fuel reprocessing plant.
